# EUROPEAN PATENT SPECIFICATION

(11) **EP 3 388 041 B1**
(45) Date of publication and mention of the grant of the patent: **04.01.2023**
(21) Application number: 15910162.5
(22) Date of filing: 11.12.2015
(51) Int. Cl.: A61G 13/00, A61N 5/10, B25J 9/06, B25J 13/08

(54) **ROBOTIZED BED**
ROBOTISIERTES BETT
LIT ROBOTISÉ

(43) Date of publication of application: 17.10.2018
(73) Proprietor: Medicaroid Corporation, Kobe-shi, Hyogo 650-0047 (JP)
(72) Inventor: HIRATSUKA, Mitsuichi, Chuo-ku Kobe 650-0047 (JP); NAKANISHI, Tetsuya, Chuo-ku Kobe 650-0047 (JP); KITANO, Yukihiko, Chuo-ku Kobe 650-0047 (JP); YANO, Yutaro, Chuo-ku Kobe 650-0047 (JP)
(74) Representative: Hoffmann Eitle
(86) International application number: PCT/JP2015/006208
(87) International publication number: WO 2017/098544

(56) References cited:
- WO-A1-2009/036169
- JP-A- H07 185 024
- JP-A- 2005 209 061
- JP-A- 2006 006 589
- JP-A- 2008 539 963
- JP-A- 2010 099 362
- JP-A- 2010 537 783
- JP-A- 2012 011 498
- US-A1- 2005 228 255
- US-A1- 2015 327 818

## Description

### TECHNICAL FIELD

The present invention relates to a robotized bed for medical purposes.

### BACKGROUND ART

In recent years, a robotized bed supporting a bed or a table by a robot arm is being introduced for medical use, and it is used for efficiency in a medical field.

Such robotized beds are used, for example, for radiation therapy (see, e.g., JP 2009-131718 A) and for angiography (see, e.g., US 8,548,629). In many cases, the robotized beds used for such purposes are intended to achieve accurate positioning of a patient during treatment and imaging.
Further related prior art may be found in US 2015/327818 A1, which refers to a positioning device for patients.

### SUMMARY OF THE INVENTION

### TECHNICAL PROBLEM

Many of such robotized beds used for medical purposes utilize verticallyarticulated robotic arms, and are large in size. It is advantageous, however, that the robotized beds for use in medial spaces, where only a limited space is available, be small in size and have a space-saving structure.

In view of the foregoing, it is therefore an object of the present invention to provide a compact robotized bed with a space-saving structure capable of being introduced even in a limited medical space.

### SOLUTION TO THE PROBLEM

The present invention is defined by the appended independent claim 1. The dependent claims describe optional features and preferred embodiments. The present invention provides a robotized bed including: a robotic arm which includes two movable elements each having a shape that extends in a particular direction and coupled together at their end portions by a horizontally-rotating joint, and a base; and a table which is in a shape that extends in a particular direction, and rotatably supported by a distal end of the robotic arm, wherein the robotized bed is configured so that the table and the robotic arm do not come in contact with each other, when the table is rotated while being maintained parallel to a horizontal plane in a state in which the robotic arm is in any posture, and the robotized bed is configured to take a posture in which the robotic arm is hidden in its entirety under the table when viewed from vertically above.

The robotized bed having a compact configuration may be introduced even in a limited space, and does not constitute an obstacle during surgery, for example.

### ADVANTAGES OF THE INVENTION

According to the present invention, efficiencies in the medical settings may be improved through the introduction of a robotized bed used for medical purposes which is downsized to have a structure that does not constitute an obstacle during medical practice, such as surgery.

### BRIEF DESCRIPTION OF THE DRAWINGS

[FIG. 1] FIG. 1 is a side view of a first configuration example of a robotic arm.
[FIG. 2] FIG. 2 is a conceptual diagram in which an actuator, a positioning device and a brake function are configured as a single unit.
[FIG. 3] FIG. 3 is a side view of an example configuration having a minimum number of degrees of freedom according to the first configuration example of the robotic arm.
[FIG. 4] FIG. 4 is a plan view of a medical room where the first configuration example of the robotic arm is disposed, and shows a state in which the table is located at a placement position.
[FIG. 5] FIG. 5 is a plan view of the medical room where the first configuration example of the robotic arm is disposed, and shows a state in which the table is located at an inspection preparation position.
[FIG. 6] FIG. 6 is a plan view of the medical room where the first configuration example of the robotic arm is disposed, and shows a state in which the table is located at an inspection position.
[FIG. 7] FIG. 7 is a side view of a second configuration example of the robotic arm.
[FIG. 8] FIG. 8 is a perspective view of the second configuration example of the robotic arm when the table is located at an MRI scanning position.
[FIG. 9] FIG. 9 is a perspective view of a third configuration example of the robotic arm.
[FIG. 10] FIG. 10 is a side view of the third configuration example of the robotic arm.
[FIG. 11] FIG. 11 is a side view of a variation of the third configuration example of the robotic arm.
[FIG. 12] FIG. 12 is a side view of an example configuration having a minimum number of degrees of freedom according to the third configuration example of the robotic arm.
[FIG. 13] FIG. 13 is a plan view of a medical room where the third configuration example of the robotic arm is disposed, and shows a state in which the table is located at the placement position.
[FIG. 14] FIG. 14 is a plan view of the medical room where the third configuration example of the robotic arm is disposed, and shows a state in which the table is in the process of moving to the inspection position.
[FIG. 15] FIG. 15 is a plan view of the medical room where the third configuration example of the robotic arm is disposed, and shows a state in which the table is located at the inspection position.
[FIG. 16] FIG. 16 is a perspective view of a fourth configuration example of the robotic arm.
[FIG. 17] FIG. 17 is a side view of the fourth configuration example of the robotic arm.
[FIG. 18] FIG. 18 is a side view of an example configuration having a minimum number of degrees of freedom according to the fourth configuration example of the robotic arm.
[FIG. 19] FIG. 19 is a plan view of a medical room where the fourth configuration example of the robotic arm is disposed, and shows a state in which the table is located at the placement position.
[FIG. 20] FIG. 20 is a plan view of the medical room where the fourth configuration example of the robotic arm is disposed, and shows a state in which the table is in the process of moving to the inspection position.
[FIG. 21] FIG. 21 is a plan view of the medical room where the fourth configuration example of the robotic arm is disposed, and shows a state in which the table is located at the inspection position.
[FIG. 22] FIGS. 22A and 22B show an example slide mechanism used in a fifth configuration example of the robotic arm.
[FIG. 23] FIGS. 23A and 23B show an example slide mechanism used in the fifth configuration example of the robotic arm, and is capable of being controlled to slide by the actuation of an actuator.
[FIG. 24] FIG. 24 is a plan view of a medical room where the fifth configuration example of the robotic arm is disposed, and shows a state in which the table is located at the placement position.
[FIG. 25] FIG. 25 is a plan view of the medical room where the fifth configuration example of the robotic arm is disposed, and shows a state in which the table is located at inspection preparation position.
[FIG. 26] FIG. 26 is a plan view of the medical room where the fifth configuration example of the robotic arm is disposed, and shows a state in which a slide plate is located at the inspection position.
[FIG. 27] FIG. 27 is a side view of another example according to the fifth configuration example of the robotic arm.
[FIG. 28] FIG. 28 is a plan view of a medical room where another example according to the fifth configuration example of the robotic arm is disposed, and shows a state in which the table is located at the placement position.
[FIG. 29] FIG. 29 is a plan view of the medical room where another example according to the fifth configuration example of the robotic arm is disposed, and shows a state in which the table is in the process of moving to the inspection preparation position.
[FIG. 30] FIG. 30 is a plan view of the medical room where another example according to the fifth configuration example of the robotic arm is disposed, and shows a state in which the table that has arrived at the inspection preparation position is slid by the slide mechanism and then arrives at the inspection position.
[FIG. 31] FIGS. 31A-31C show examples in which the robotic arm is controlled by a warpage correction function.
[FIG. 32] FIGS. 32A-32C show other examples in which the robotic arm is controlled by the warpage correction function.
[FIG. 33] FIG. 33 is a perspective view of an MRI apparatus.
[FIG. 34] FIG. 34 is a perspective view illustrating a case where another example according to the fifth configuration example of the robotic arm is applied to an intraoperative MRI, and shows a state in which the table is located at the treatment position.
[FIG. 35] FIG. 35 is a perspective view illustrating a case where another example according to the fifth configuration example of the robotic arm is applied to the intraoperative MRI, and shows a state in which the table is located at the MRI scanning preparation position.
[FIG. 36] FIG. 36 is a perspective view illustrating a case where another example according to the fifth configuration example of the robotic arm is applied to the intraoperative MRI, and shows a state in which the table is located at the MRI scanning position.
[FIG. 37] FIG. 37 is a perspective view illustrating a case where the fourth configuration example of the robotic arm is combined with an angiographic apparatus, and shows a state before the table is inserted in the C-shaped arm of the angiographic apparatus.
[FIG. 38] FIG. 38 is a perspective view illustrating a case where the fourth configuration example of the robotic arm is combined with the angiographic apparatus, and shows a state after the table is inserted in the C-shaped arm of the angiographic apparatus.
[FIG. 39] FIG. 39 is a perspective view illustrating a case where the fourth configuration example of the robotic arm is combined with a surgical robot.
[FIG. 40] FIG. 40 is a block diagram illustrating a configuration of a controller.

### DESCRIPTION OF EMBODIMENTS

In medical settings, efforts have been made to improve the medical settings for more efficient and accurate treatment, inspection, measurement, etc., while maintaining safety in various scenes. The present invention suggests introducing, into the medical settings, a robotized bed whose table, on which a target is to be placed, is supported by a robotic arm having multiple degrees of freedom (i.e., three or more degrees of freedom), thereby enhancing the efficiency and accuracy in the treatment, inspection, measurement, etc.

### [Configuration of Robotized Bed]

### (First Configuration Example)

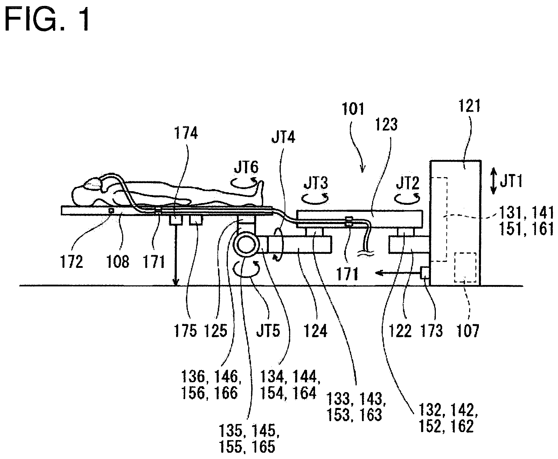

FIG. 1 shows a side view of a robotized bed according to a first configuration example of the present invention. A robotic arm 101 for use in this robotized bed has multiple degrees of freedom (i.e., three or more degrees of freedom), and has a distal end supporting a table 108 on which a target is placed. The table 108 and the robotic arm 101 form the robotized bed.

As shown in FIG. 1, the robotic arm 101 includes a base 121, a plurality of movable elements (first to fourth movable elements 122-125 in the present configuration example) and a plurality of joints (first to sixth joints 131-136 in the present configuration example).

The base 121 and one end portion of the first movable element 122 are coupled together by the first joint 131 which is a linearly movable joint in a vertical direction. The first joint 131 enables the movable element 122 to move in a first axial direction (i.e., in a vertical direction). The other end portion of the first movable element 122 and one end portion of the second movable element 123 are coupled together by a horizontally-rotating joint, which enables the movable element 123 to rotate about a second axis (the vertical direction). The other end portion of the second movable element 123 and one end portion of the third movable element 124 are coupled together by a horizontally-rotating joint, which enables the third movable element 124 to rotate about a third axis (the vertical direction). The fourth to sixth joints 134-136 between the third movable element 124 and the fourth movable element 125 are rotating joints which rotate about fourth to sixth axes, respectively. The direction of the fourth axis corresponds to a longitudinal direction of the third movable element 124. The fifth axis corresponds to a direction orthogonal to the fourth axis and is rotated by the fourth joint 134. The sixth axis corresponds to a direction orthogonal to the fifth axis and is rotated by the fifth joint 135. Note that in FIG. 1 the movement directions of the first to sixth joints 131-136 are indicated by arrows JT1-JT6, respectively.

Each of the second movable element 123 and the third movable element 124 is a rod-like member extending in a particular direction, with its length appropriately designed according to a required range of movement of the robotic arm 101. The "one end portion" of a movable element extending in a particular direction refers to either one of the two end regions when the movable element is equally divided into three regions in the particular direction (i.e., the longitudinal direction). The "other end portion" of the movable element extending in the particular direction refers to the end portion opposite to the other end portion of the two end regions of the three equally-divided regions of the movable element in the particular direction (i.e., the longitudinal direction). If it is simply called the "end portion," it refers to either the one end portion or the other end portion. The portion between both of the two end portions is called a "middle portion."

The fourth movable element 125 is provided at the distal end of the robotic arm 101. In the present configuration example, the distal end of the robotic arm 101 is fixed on a lower surface of one end portion of the table 108 extending in a particular direction.

The robotic arm 1A includes a plurality of actuators (first to sixth actuators 141-146 in the present configuration example) associated with the first to sixth joints 131-136 to move or rotate the first to fourth movable elements 122-125, a plurality of position detectors (first to sixth position detectors 151-156 in the present configuration example) built in the respective joints to detect the positions of the respective movable elements, and a controller 107 (see FIG. 1) which controls the actuation of the respective actuators. The controller 107 is provided in the base 121, but may also be an independent external device, for example.

The first to sixth actuators 141-146 may be servo motors, for example. Encoders which detect a rotation angle or a direction of a motor are generally used as the position detectors, but resolvers or potentiometers may also be used as the position detectors.

It is recommended that the robotic arm 101 further include first to sixth electromagnetic brakes 161-166 associated with the first to sixth joints 131-136, respectively. If the robotic arm 101 does not include any electromagnetic brakes, the posture of the robotic arm 101 is maintained by actuating the plurality of actuators 141-146. If the robotic arm 101 includes the electromagnetic brakes, the posture of the robotic arm 1A may be maintained by turning the electromagnetic brakes on even if some of the actuators are turned off.

In the case where the electromagnetic brakes are provided, each of the first to sixth electromagnetic brakes 161-166 is configured to turn its brake function on when no drive current is supplied to the associated one of the actuators, and to turn its brake function off when a drive current is supplied to the actuator.

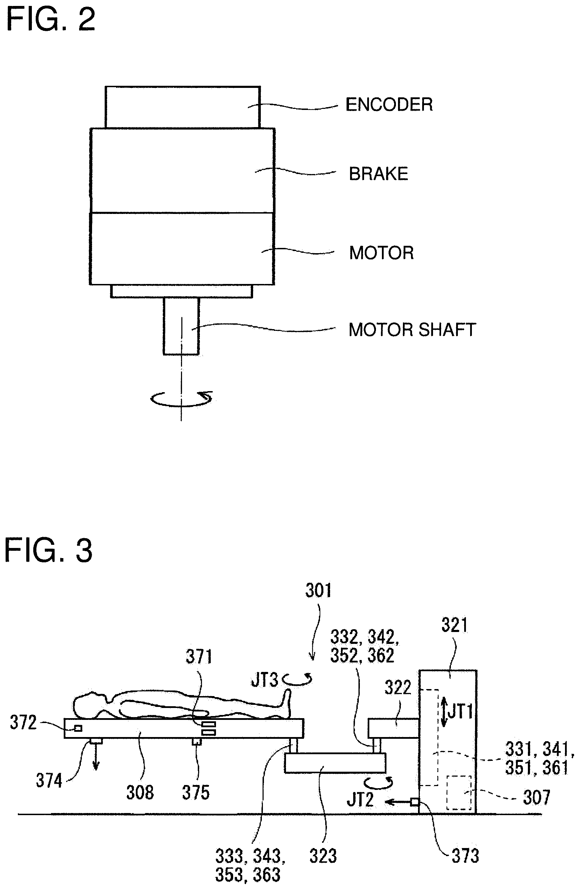

In many cases, a motor functioning as the actuator, an encoder functioning as the position detector, and the brake are integrated together as a unit as shown in FIG. 2. Further, each of the first to sixth actuators 141-146 is provided with a deceleration mechanism for power transmission, a coupling, etc.

In the above example, the robotic arm 101 shown in FIG. 1 has 6 degrees of freedom. However, the degrees of freedom of the robotic arm of the present invention do not have to be 6, but may be 5 or less or 7 or more. It is nevertheless recommended that the degrees of freedom of the robotic arm be 3 or more so that the table 108 can move at least in a straight manner in the room. FIG. 3 shows an example robotized bed having 3 degrees of freedom. In FIG. 3, the robotic arm 301 is comprised of a base 321 and two movable elements 322 and 323. The base 321 and one end portion of the first movable element 322 are coupled together by a first joint 331 traveling vertically straight, which enables the first movable element 322 to move in a first axial direction (in a vertical direction). The other end portion of the first movable element 322 and one end portion of the second movable element 323 are coupled together by a horizontally-rotating joint, which enables the second movable element 323 to rotate about a second axis (the vertical direction). The other end portion of the second movable element 323 serves as the distal end of the robotic arm 301, and is coupled to one end portion of the table 308 by a horizontally-rotating joint.

The robotized bed having the above configurations makes it possible to move the table, on which the target has been placed, to a target position, such as an inspection position and a treatment position, accurately and quickly, thus achieving significant improvement in the efficiency of the inspection and treatment in medical settings. For example, compared to the configuration in which a table with a caster is used to move the patient, the table may be moved more smoothly without shaking the patient too much, and may be prevented from being tangled with a lot of cords of medical equipment and the tubes of medical instruments which run on the floor of the medical room, and can be prevented from being wobbled by stepping over the cords and tubes. This thus improves safety and transfer efficiency.

Examples of the target positions of the robotized bed include: a placement position where a target, such as a human being and an animal, is placed on the robotized bed; an inspection position where an inspection is conducted using specific inspection equipment or measurement equipment; an imaging position where an image of a specific site of the placed target is taken by CT, MRI, angiography, etc.; a treatment preparation position where a nurse or other staff give medical attention to the patient before treatment; and a treatment position (including the surgical position) where a doctor and an assistant give treatment (including surgery). The robotized bed may be moved to different positions even for the same purpose, if, for example, different treatments need to be given at a plurality of sites. Specifically, the robotized bed may be used, for example, as follows: the table may be moved to the inspection position to inspect the placed target for any objects like an implant which affect MRI, before being moved to the MRI scanning position; the table may be moved to the inspection position to detect an amount of radioactive substances deposited using a detector, before the patient, who is a placed target, is moved to the surgical position; the patient, who is a placed target, may be moved to the inspection position to check his/her skin condition, before the patient is moved to the surgical position for skin surgery; and the table may be moved to the imaging position for brain tomography by an MRI apparatus, before being moved to the surgical position for surgery removing a brain tumor.

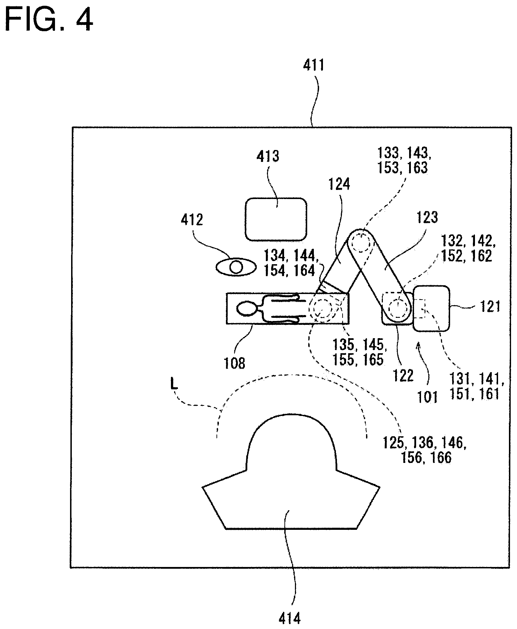

The movements of the table 108 supported by the robotic arm 101 of the present example between the plurality of positions will be described with reference to FIGS. 4-6.

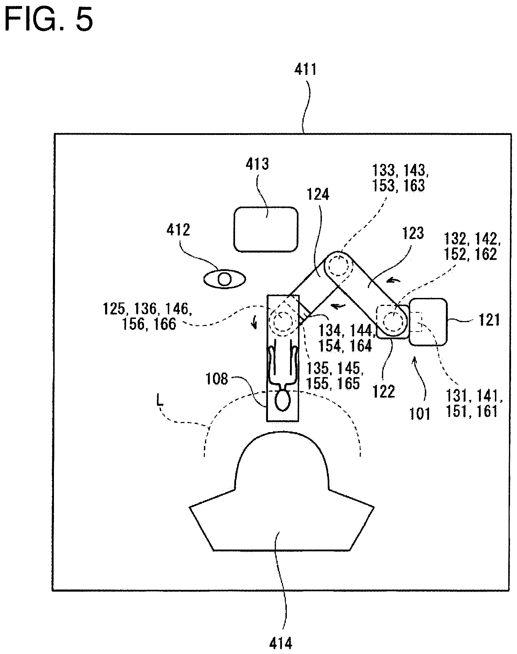

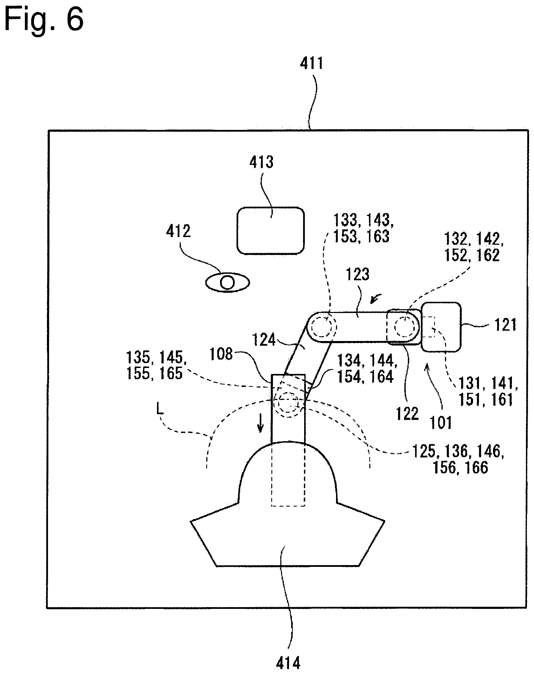

FIG. 4 shows a state in which the table 108 is located at the placement position in the process of moving a subject, who is a placed target, from the placement position to the inspection position where an inspection will be conducted by an inspection device. FIG. 5 shows a state in which the second movable element 123 and the third movable element 124 are moved by the controller 107 as the arrows indicate, and the table 108 is rotated about the sixth axis as the arrow indicates (in some cases, the first movable element 122, too, is moved in the vertical direction to have its height adjusted, and the table is rotated about the fourth axis and/or the fifth axis to have its tilt finely adjusted), causing the head of the subject to be directed toward the inspection device 414. FIG. 6 shows a state in which the table 108 is inserted in the inspection device 414, and the subject has arrived at the inspection position. Note that the position of the table 108 shown in FIG. 4 may also be the treatment position. From the inspection position shown in FIG. 6, the respective movable elements move in reverse direction until the table 108 returns to the position shown in FIG. 4, where a doctor 412 can give a treatment based on the result of the inspection that has just been conducted.

The movement of the table 108 by the robotic arm 101 between the respective positions may be achieved by, for example, giving an instruction to move the movable elements of the robotic arm 101 to the controller 107 through a teaching pendant. Alternatively, the respective positions, such as the treatment position and the inspection position, may be stored in the controller 107 in advance. In this configuration, giving, for example, only a forward-movement instruction to the controller makes the movable elements work in such a manner that moves the table 108 to the target position in the shortest time. The table 108 can thus be moved to the target position more quickly and smoothly. Further, the target position and some points on the intended path to the target position may be designated. In this configuration, the table 108 may automatically travel along the intended path and arrive at the target position simply by giving, for example, a movement start instruction to the controller 107. To record the respective positions, the respective positions may be directly stored by actually guiding the robotic arm 101 to the target position through the teaching pendant. Alternatively, the respective positions may also be designated by inputting their x, y and z coordinates.

### (Second Configuration Example)

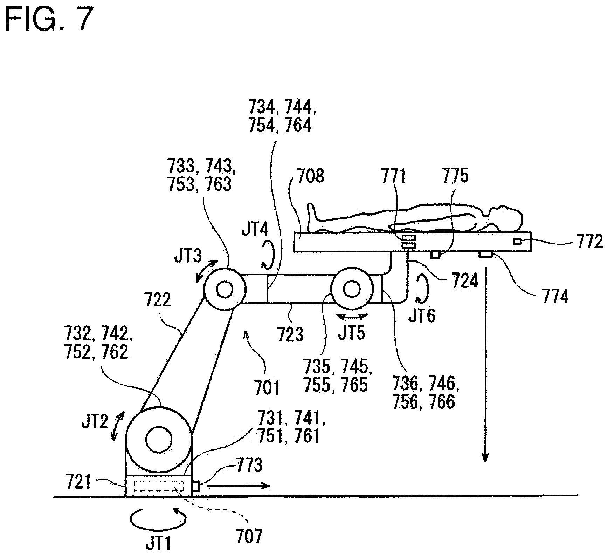

FIG. 7 shows a side view of a robotized bed according to a second configuration example of the present invention. A robotic arm 701 for use in this robotized bed is a socalled vertically articulated robotic arm having multiple degrees of freedom (i.e., three or more degrees of freedom), and has a distal end supporting a table 708 on which a target is placed. The table 708 and the robotic arm 701 form the robotized bed.

As shown in FIG. 7, the robotic arm 701 includes a plurality of movable elements (first to third movable elements 722-724 in the present embodiment) and a plurality of joints (first to sixth joints 731-736 in the present embodiment).

A base 721 has a horizontally-rotating joint which rotates about a first axis (a vertical direction). The base 721 and one end portion of the first movable element 722 are coupled together by a vertically-rotating joint 732 which rotates about a second axis orthogonal to the first axis. The other end portion of the first movable element 722 and one end portion of the second movable element 723 are coupled together by a vertically-rotating joint which rotates about a third axis that is parallel to the second axis. The second movable element 723 is a rod-like member extending in a particular direction, is rotated by the third axis, and has a rotating joint 734 that is rotatable about a fourth axis which coincides with the particular direction. The other end portion of the second movable element 723 is coupled to one end portion of the third movable element 724 by a vertically-rotating joint 735 which rotates about a fifth axis that is orthogonal to the fourth axis. The third movable element 724 also has a rotating joint 936 that is rotatable about a sixth axis which is orthogonal to the fifth axis.

Similarly to the second movable element 723, the first movable element 722 is also a rod-like member extending in a particular direction, with its length appropriately designed according to a required range of movement of the robotic arm 701.

The third movable element 724 is provided at the distal end of the robotic arm 701. In the present configuration example, the distal end of the robotic arm 701 is fixed on a lower surface of one end portion of the table 708 extending in a particular direction. The area where the distal end of the robotic arm supports the table 708 may be located at an end portion or a middle portion of the table 708. The definitions of the "one end portion," "other end portion," "end portion" and "middle portion" are the same as, or similar to, those in the first configuration example.

The robotic arm 701 includes a plurality of actuators (first to sixth actuators 741-746 in the present configuration example) associated with the first to sixth joints 731-736 to move or rotate the first to third movable elements 722-724, a plurality of position detectors (first to sixth position detectors 751-756 in the present configuration example) built in the respective joints to detect the positions of the respective movable elements, and a controller 707 (see FIG. 7) which controls the actuation of the respective actuators. The controller 707 is provided in the base 721, but may also be an independent external device, for example.

The first to sixth actuators 741-746 may be servo motors, for example. Similarly to the first configuration example, encoders, resolvers or potentiometers may be used as the position detectors.

It is recommended that the robotic arm 701 further include first to sixth electromagnetic brakes 761-766 associated with the first to sixth joints 731-736, respectively. If the robotic arm 701 does not include any electromagnetic brakes, the posture of the robotic arm 701 is maintained by actuating the plurality of actuators 741-746. If the robotic arm 701 includes the electromagnetic brakes, the posture of the robotic arm 701 may be maintained by turning the electromagnetic brakes on even if some of the actuators are turned off.

In the case where the electromagnetic brakes are provided, each of the first to sixth electromagnetic brakes 761-766 is configured to turn its brake function on when no drive current is supplied to the associated one of the actuators, and to turn its brake function off when a drive current is supplied to the actuator.

Similarly to the first configuration example, in many cases, a motor functioning as the actuator, an encoder functioning as the position detector, and the brake are integrated together as a unit as shown in FIG. 2. Further, each of the first to sixth actuators 741-746 is provided with a deceleration mechanism for power transmission, a coupling, etc.

The robotic arm 701 shown in FIG. 7 has 6 degrees of freedom. However, the degrees of freedom of the robotic arm of the present invention do not have to be 6, but may be 5 or less or 7 or more. It is nevertheless recommended that the degrees of freedom of the robotic arm be 3 or more so that the table 708 can move at least in a straight manner in the room.

The robotized bed having the above configurations makes it possible to move the table, on which the target has been placed, to a target position, such as an inspection position and a treatment position, accurately and quickly, thus achieving significant improvement in the efficiency of the inspection and treatment in medical settings. For example, compared to the configuration in which a table with a caster is used to move the patient, who is a placed target, the table 708 may be moved more smoothly without shaking the patient too much, and may be prevented from being tangled with a lot of cords of medical equipment and the tubes of medical instruments which run on the floor of the medical room, and may be prevented from being wobbled by stepping over the cords and tubes. This thus improves safety and transfer efficiency.

Examples of the target positions of the robotized bed are the same as, or similar to, those described in the first configuration example, and description thereof will be omitted here.

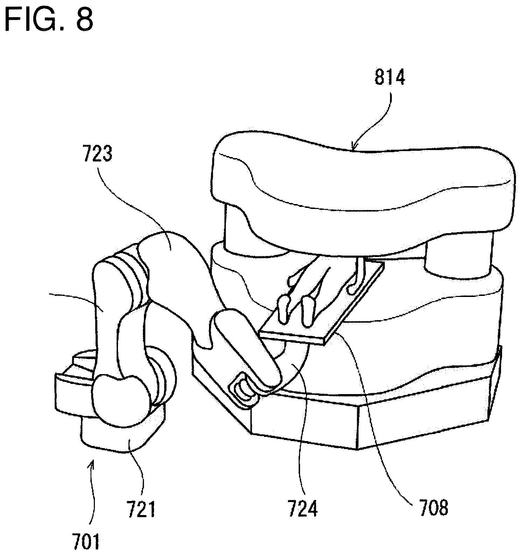

The robotic arm 701 according to the present configuration example, too, is capable of moving the table between the plurality of positions along arbitrary paths as long as the paths are within a range of movement of the robotic arm 701. Thus, the table may be moved to the inspection device or any other positions by following the same paths described in the first configuration example shown in FIGS. 4-6. Shown in FIG. 8 for reference is a perspective view of a state in which the placed target is a human being to be imaged and the target position is the MRI scanning position, and in which the table has moved from the placement position and arrived at the MRI scanning position.

### (Third Configuration Example)

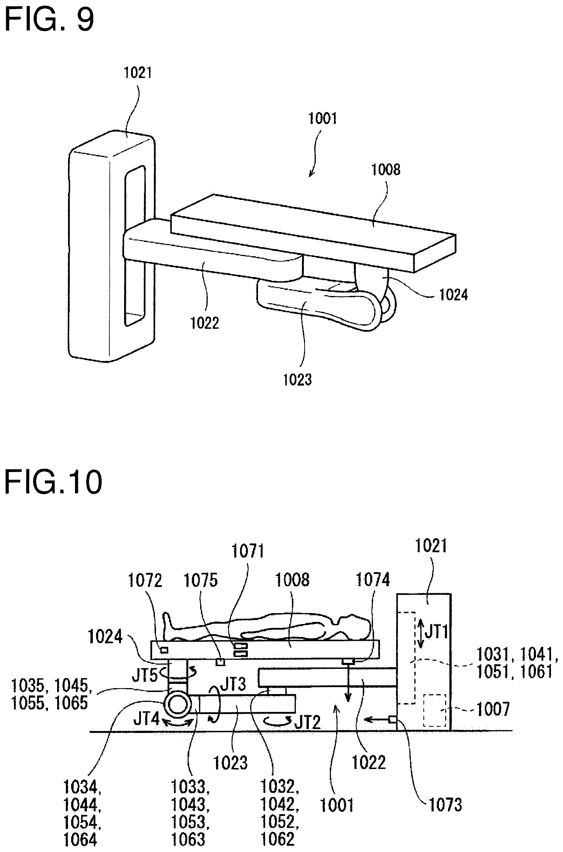

An appearance and a side view of a robotized bed according to a third configuration example of the present invention are shown in FIG. 9 and FIG. 10, respectively. A robotic arm 1001 for use in this robotized bed has multiple degrees of freedom (three or more degrees of freedom), and has a distal end supporting a table 1008 on which a target is placed. The table 1008 and the robotic arm 1001 form the robotized bed.

As shown in FIG. 10, the robotic arm 1001 includes a base 1021, a plurality of movable elements (first to third movable elements 1022-1024 in the present configuration example), and a plurality of joints (first to fifth joints 1031-1035 in the present configuration example).

The base 1021 and one end portion of the first movable element 1022 are coupled together by the first joint 1031 traveling vertically straight, which enables the first movable element 1022 to move in a first axial direction (in a vertical direction). The other end portion of the first movable element 1022 and one end portion of the second movable element 1023 are coupled together by a horizontally-rotating joint, which enables the second movable element 1023 to rotate about a second axis (the vertical direction). The third to fifth joints 1033-1035 between the second movable element 1023 and the third movable element 1024 are joints rotating about third to fifth axes, respectively. The third axis corresponds to a direction in which the second movable element 1023 extends. The fourth axis corresponds to a direction orthogonal to the third axis about which the third joint 1033 rotates. The fifth axis corresponds to a direction orthogonal to the fourth axis about which the fourth joint 1034 rotates.

Each of the first movable element 1022 and the second movable element 1023 is a rod-like member extending in a particular direction, with its length appropriately designed according to a required range of movement of the robotic arm 1001. The first movable element 1022 moves up and down, while staying parallel to the horizontal plane, and the second movable element 1023 rotates about the second axis, while staying parallel to the first movable element 1022. This configuration does not require the second actuator 1042 to compensate for the gravity in the vertical direction, and the motor may thus be reduced in size. This is advantageous in downsizing the robotic arm 1001, and is advantageous in introducing the robotic arm 1001 in the medical settings where only a limited space is available, or in giving a larger space for treatments and surgery.

Further, the robotized bed of the present configuration example is configured such that the table 1008 does not contact with the robotic arm 1001, no matter how much (e.g., by 360 degrees) the table 1008 is rotated while keeping table 1008 parallel to the horizontal plane, in a state in which particular directions (i.e., the longitudinal directions) of the first movable element 1022 and the second movable element 1023, which are coupled together at their end portions by a horizontally-rotating joint, are parallel to each other when viewed from vertically above. Specifically, the robotized bed of the present configuration example is configured such that, in a state in which the first movable element 1022 and the second movable element 1023, which are coupled together at their end portions by a horizontally-rotating joint, and the table 1008 are arranged parallel to the horizontal plane, the table 1008 is not level with the other movable elements and is located at the top. In other words, in a state in which the distal end of the robotic arm 1001 is located at the lowermost position of its motion range and the table 1008 takes a position parallel to the horizontal plane, the first and second movable elements of the robotic arm 1001 are lower than the lower surface of the table 1008. Further, in the present configuration example, the base 1021 is higher than the lower surface of the table 1008 in order to provide a greater range of adjustment for the vertical movement of the table 1008, even in a state in which the distal end of the robotic arm 1001 is located at the lowermost position of its motion range and the table 1008 takes a position parallel to the horizontal plane. These configurations allow the movable elements of the robotic arm 1001 to be located and housed under the table 1008, and hence allow effective use of a limited space in the medical settings while ensuring a sufficiently broad range for the vertical movement of the table 1008.

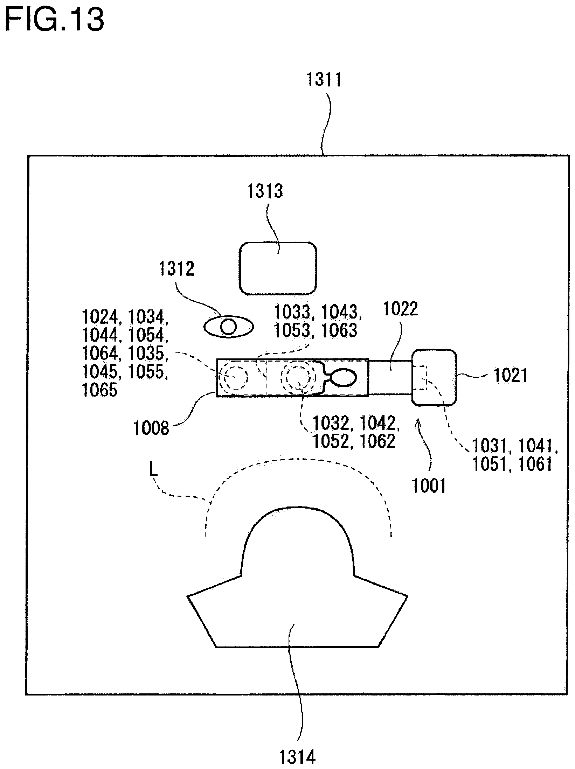

This advantage can be seen clearly from FIGS. 13-15 illustrating the movement of the robotized bed according to the third configuration example. As shown in FIG. 13, the robotized bed of the present configuration example may take a position in which the respective movable elements and the table 1008 overlap one another when viewed from vertically above. On the other hand, the robotized beds of the first and second configuration examples have difficulty in taking the same or similar position as the position shown in FIG. 13 where the table is brought as close to the base as possible to ensure a treatment space. Specifically, in the first configuration example, the second movable element 123 and the third movable element 124 cannot be positioned under the table 108 and become an obstacle as shown in FIG. 4. In the second configuration example, the table 708 may be positioned higher, in theory, than the respective movable elements if the table 708 is raised to a very high level. Actually, however, positioning the table 708 at such a high level causes inconvenience in giving treatment and inspection and placing a target on the table, and is therefore impractical. As mentioned earlier, vertically articulated robotic arms require compensation for gravity, and hence require an actuator large in size. It is therefore difficult to position the respective movable elements under the table 708 while supporting the table 708 from under the table 708, as can be seen from the conceptual diagram shown in FIG. 8.

Further, it is recommended that the width of the table 1008 is greater than the width of each of the movable elements of the robotic arm 1001. For example, it is beneficial that in a state in which particular directions (i.e., the longitudinal directions) of the first movable element 1022 and the second movable element 1023, which are coupled together at their end portions by a horizontally-rotating joint, and a particular direction (i.e., the longitudinal direction) of the table 1008 are parallel to one another when viewed from vertically above, the first movable element 1022 and the second movable element 1023 be hidden under the table 1008 in the direction orthogonal to the particular direction at portions where the table 1008 overlaps with the first movable element 1022 and the second movable element 1023 in the particular direction (i.e., the longitudinal direction) when viewed from vertically above. This configuration allows parts of the robotic arm 1001 (that is, in the example of FIG. 10, all of the first movable element 1022 except the one end portion thereof, and all of the second movable element 1023 and third movable element 1024) which overlap with the table 1008 in the longitudinal direction of the table 1008, to be housed under the table 1008 at least in the width direction of the table 1008 (i.e., the direction orthogonal to the particular direction in which the table 1008 extends) (see, e.g., FIG. 13).

In the examples shown in FIGS. 9 and 10, one (i.e., the first movable element 1022) of the two movable elements (namely, the first movable element 1022 and the second movable element 1023) which are coupled together at their end portions by a horizontally-rotating joint is directly coupled to the base 1021. However, the movable element may also be indirectly coupled to the base via another horizontally-rotating joint or a vertically-rotating joint. In this case, as well, the advantages of ensuring a larger space and downsizing the robotic arm are achieved, as long as the above-described positional relationship is maintained and the plurality of the movable elements are housed under the table 1008.

The third movable element 1024 is provided at the distal end of the robotic arm 1001. In the present configuration example, the distal end of the robotic arm 1001 is fixed on a lower surface of one end portion of the table 1008 extending in the particular direction. This configuration allows the robotic arm 1001 to move such that the other end portion of the table 1008 is positioned as far away from the base 1021 as possible. Supporting the table 1008 at its one end portion increases the movable range of the table 1008, but the table 1008 may be supported at its middle portion if a priority is placed on the supporting strength.

The definitions of the "one end portion," "other end portion," "end portion" and "middle portion" as adopted in the above description are the same as, or similar to, those adopted in the first and second configuration examples.

The robotic arm 1001 includes a plurality of actuators (first to fifth actuators 1041-1045 in the present configuration example) associated with the first to fifth joints 1031-1035 to move or rotate the first to third movable element 1022-1024, a plurality of position detectors (first to fifth position detectors 1051-1055 in the present configuration example) built in the respective joints to detect the positions of the respective movable elements, and a controller 1007 (see FIG. 10) which controls the actuation of the respective actuators. The controller 1007 is provided in the base 1021, but may also be an independent external device, for example.

The first to fifth actuators 1041-1045 are servo motors, for example. Similarly to the first and second configuration examples, encoders, resolvers and potentiometers may be used as the position detectors.

It is recommended that the robotic arm 1001 further includes first to fifth electromagnetic brakes 1061-1065 associated with the first to fifth joints 1031-1035, respectively. If the robotic arm 1001 does not include any electromagnetic brakes, the posture of the robotic arm 1001 is maintained by actuating the plurality of actuators 1041-1045. If the robotic arm 1001 includes the electromagnetic brakes, the posture of the robotic arm 1001 may be maintained by turning the electromagnetic brakes on even if some of the actuators are turned off.

In the case where the electromagnetic brakes are provided, each of the first to fifth electromagnetic brakes 1061-1065 is configured to turn its brake function on when no drive current is supplied to the associated one of the actuators, and to turn its brake function off when a drive current is supplied to the actuator.

Similarly to the first and second configuration examples, in many cases, a motor functioning as the actuator, an encoder functioning as the position detector, and the brake are integrated together as a unit as shown in FIG. 2. Further, each of the first to fifth actuators 1041-1045 is provided with a deceleration mechanism for power transmission, a coupling, etc.

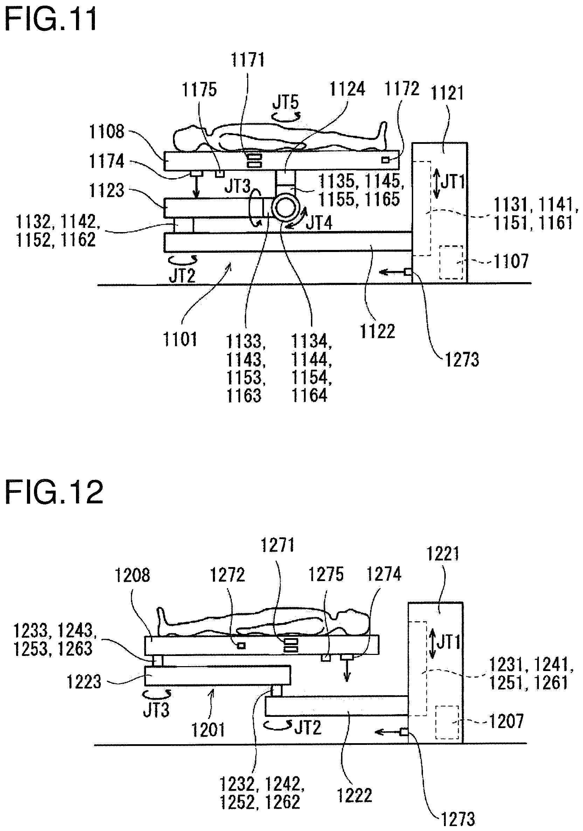

In the example shown in FIG. 10, the first movable element 1022 is coupled by the horizontally-rotating joint 1032 so as to be located above the second movable element 1023. Shown in FIG. 11 as a variation of the present configuration example is a robotic arm 1101, of which the first movable element 1122 is coupled by a horizontally-rotating joint 1132 so as to be located below the second movable element 1123.

In the present variation, the base 1121 and one end portion of the first movable element 1122 are coupled together by a first joint 1131 traveling vertically straight, which enables the first movable element 1122 to move in a first axial direction (in a vertical direction). The other end portion of the first movable element 1122 and one end portion of the second movable element 1123 are coupled together by a horizontally-rotating joint, which enables the second movable element 1123 to rotate about a second axis (the vertical direction) above the first movable element 1122. Third to fifth joints 1133-1135 between the second movable element 1123 and the third movable element 1124 are rotating joints which rotate about third to fifth axes, respectively. The third axis corresponds to a direction in which the second movable element 1123 extends. The fourth axis corresponds to a direction orthogonal to the third axis about which the third joint 1133 rotates. The fifth axis corresponds to a direction orthogonal to the fourth axis about which fourth joint 1134 rotates.

The third movable element 1124 is provided at the distal end of the robotic arm 1101. In the present configuration example, the distal end of the robotic arm 1101 is fixed on a lower surface of a middle portion of the table 1108 extending in the particular direction. This configuration allows supporting the table 1108 while placing a priority on the supporting strength. Naturally, the table 1108 may be supported at its one end portion to place a priority on the movable range of the table 1108. In that case, however, it is necessary to determine the lengths of the respective movable elements 1122-1124 and the table 1108 appropriately in order to avoid contact with the robotic arm 1101 even when the table 1108 is freely rotated while staying parallel to the horizontal plane.

The robotic arms 1001•1101 shown in FIGS. 10 and 11 have 5 degrees of freedom. However, the degrees of freedom of the robotic arm of the present invention do not have to be 5, but may be 4 or less or 6 or more. It is nevertheless recommended that the degrees of freedom of the robotic arm be 3 or more so that the table 1008•1108 can move at least in a straight manner in the room. FIG. 12 shows an example robotized bed having 3 degrees of freedom. In FIG. 12, the robotic arm 1201 is comprised of a base 1221 and two movable elements 1222 and 1223. The base 1221 and one end portion of the first movable element 1222 are coupled together by a first joint 1231 traveling vertically straight, which enables the movable element 1222 to move in a first axial direction (in a vertical direction). The other end portion of the first movable element 1222 and one end portion of the second movable element 1223 are coupled together by a second joint 1232, which is a horizontally-rotating joint enabling the movable element 1223 to rotate about a second axis (the vertical direction). The other end portion of the second movable element 1223 serves as the distal end of the robotic arm 1201, and is coupled to one end portion of the table 1208 by a third joint 1233, which is a horizontally-rotating joint.

The robotized bed having the above configurations makes it possible to move the table 1008•1108•1208, on which a target has been placed, to a target position, such as an inspection position and a treatment position, accurately and quickly, thus achieving significant improvement in the efficiency of the inspection and treatment in medical settings. For example, compared to the configuration in which a table with a caster is used to move the patient, the table 1008•1108•1208 may be moved more smoothly without shaking the patient too much, and may be prevented from being tangled with a lot of cords of medical equipment and the tubes of medical instruments which run on the floor of the medical room, and may be prevented from being wobbled by stepping over the cords and tubes. This thus improves safety and transfer efficiency.

Further, in the robotized bed according to the present configuration example, the movable elements indicated by the reference characters 1023, 1123, 1223 are coupled to the table indicated by the reference character 1208 by the joints indicated by the reference characters 1032•1132•1232•1233, each of which is a horizontally-rotating joint that enables the movable elements and the table to rotate while always staying parallel to the horizontal plane. This configuration thus provides greater stiffness, compared to the case where each of the movable elements and the table are coupled by a vertically-rotating joint. Specifically, if the movable element and the table are coupled together by a vertically-rotating joint, the posture may not be completely maintained by only the control by the actuator, and warpage may occur, due to, for example, the weight of the placed target, while the table is being moved or staying in some posture. The horizontally-rotating joint, on the other hand, does not rotate in the vertical direction, and therefore such warpage hardly occurs. Moreover, it is not necessary to take into account the rotation in the vertical direction at a point where the horizontally-rotating joint is provided which always enables rotation parallel to the horizontal plane. Thus, the electromagnetic brake may be omitted even if the situation in which the power is turned off is taken into consideration. Note that the same holds true for the horizontally-rotating joints indicated by the reference characters 132, 133, 332, 333 in the robotized bed according to the first configuration example. However, the robotized bed described in the present configuration example has greater stiffness and also contributes to providing a larger treatment space, and is designed to be more suitable as a robotized bed used in a medical room.

Examples of the target positions of the robotized bed are the same as, or similar to, those described in the first and second configuration examples, and description thereof will be omitted here.

The movements of the table 1008 supported by the robotic arm 1001 of the present configuration example between the plurality of positions will be described with reference to FIGS. 13-15.

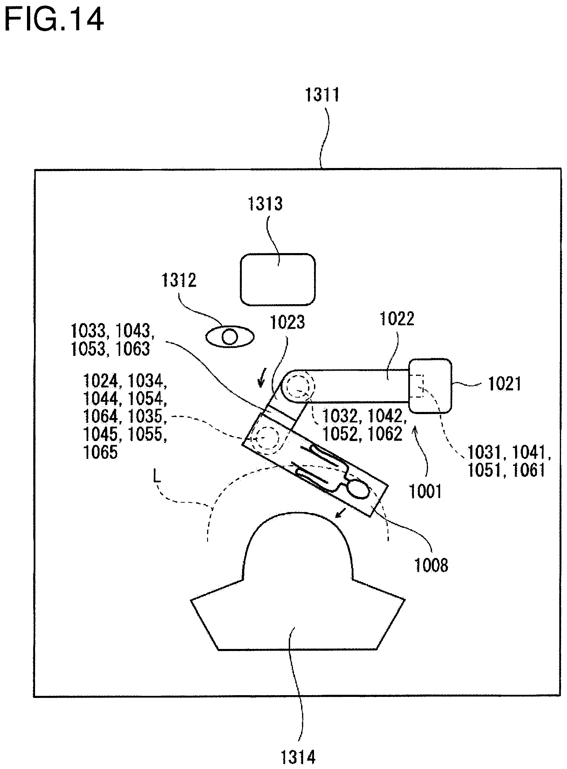

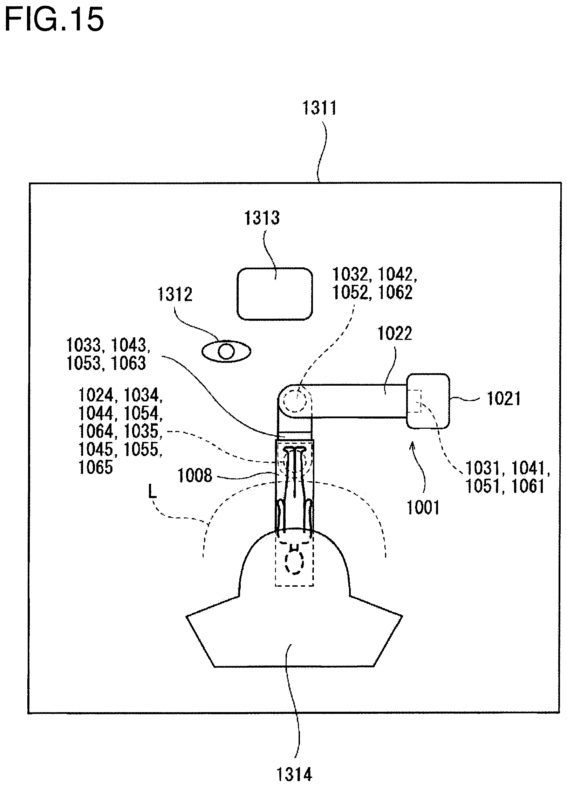

FIG. 13 shows a state in which the table 1008 is located at the placement position in the process of moving a subject, who is a placed target, from the placement position to the inspection position. FIG. 14 shows a state in which the second movable element 1023 and the table 1008 are moved by the control of the controller 1007 as the arrows indicate (in some cases, the first movable element, too, is moved in the vertical direction to have its height adjusted, and the table 1008 is rotated about the third axis and/or the fourth axis to have its tilt finely adjusted), causing the head of the subject to move toward the inspection device 1314 from an oblique angle. FIG. 15 shows a state in which the table 1008 is inserted in the inspection device 1314, and the subject has arrived at the inspection position. Note that the position of the table 1008 shown in FIG. 13 can also be the treatment position. From the inspection position shown in FIG. 15, the respective movable elements move in reverse direction until the table 1008 returns to the position shown in FIG. 13, where a doctor 1312 can give a treatment based on the result of the inspection that has just been conducted.

The robotic arm 1201 shown in FIG. 12, as well, enables the table 1208 to follow a similar path. Turning to the robotic arm 1101 shown in FIG. 11, the table 1108 can arrive at the inspection position by rotating the second movable element 1123 and the table 1108 in the direction opposite to the direction indicated by the arrows shown in FIG. 14 (in some cases, the first movable element 1122, too, moves in the vertical direction to adjust the height thereof).

How to give instructions to move the robotic arm, and how to set the target position to which the table is going to move are the same as, or similar to, what has been described in the first and second configuration examples.

### (Fourth Configuration Example)

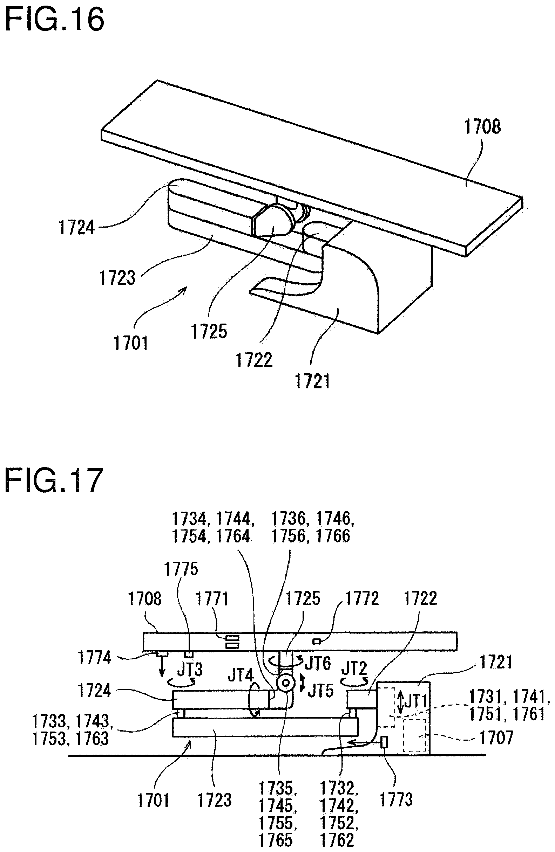

A perspective view and a side view of a robotized bed according to a fourth configuration example of the present invention are shown in FIG. 16 and FIG. 17, respectively. A robotic arm 1701 for use in this robotized bed has multiple degrees of freedom (i.e., three or more degrees of freedom), and has a distal end supporting a table 1708 on which a target is placed. The table 1708 and the robotic arm 1701 form the robotized bed.

As shown in FIG. 17, the robotic arm 1701 includes a base 1721, a plurality of movable elements (first to fourth movable elements 1722-1725 in the present configuration example), and a plurality of joints (first to sixth joints 1731-1736 in the present configuration example).

The base 1721 and one end portion of the first movable element 1722 are coupled together by the first joint 1731 traveling vertically straight, which enables the first movable element 1722 to move in a first axial direction (in a vertical direction). The other end portion of the first movable element 1722 and one end portion of the second movable element 1723 are coupled together by a horizontally-rotating joint, which enables the second movable element 1723 to rotate about a second axis (the vertical direction). The other end portion of the second movable element 1723 and one end portion of the third movable element 1724 are coupled together by a horizontally-rotating joint, which enables the third movable element 1724 to rotate about a third axis (the vertical direction) which is rotated by, and parallel to, the second axis. The fourth to sixth joints 1734-1736 between the third movable element and the fourth movable element are joints rotating about fourth to sixth axes, respectively. The fourth axis corresponds to a direction in which the third movable element 1724 extends. The fifth axis corresponds to a direction orthogonal to the fourth axis about which the fourth joint 1734 rotates. The sixth axis corresponds to a direction orthogonal to the fifth axis about which the fifth joint 1735 rotates.

Each of the second movable element 1723 and the third movable element 1724 is a rod-like member extending in a particular direction, with its length appropriately designed according to a required range of movement of the robotic arm 1701. The first movable element 1722 is configured to move up and down, while staying parallel to the horizontal plane. The second movable element 1723 and the third movable element 1724 are configured to rotate while staying parallel to the first movable element 1722. This configuration does not require the second and third actuators 1742 and 1743 to compensate for the gravity in the vertical direction, and the motor may thus be reduced in size. This is advantageous in downsizing the robotic arm 1701, and is advantageous in introducing the robotic arm 1701 in the medical settings where only a limited space is available, or in giving a larger space for treatments and surgery.

Further, in the robotized bed of the present configuration example, the height of the base 1721 is reduced instead of limiting the range of movement of the first movable element 1722 by the first joint in the vertical direction. The reduction in height of the base 1721 prevents the table 1708 from contacting with the robotic arm 1701, even when the first movable element 1722 is moved up and down (in the vertical direction) with the table 1708 maintained parallel to the horizontal plane, or no matter how much (e.g., 360 degrees) the table 1708 is rotated. Thus, in the present configuration example, the table and the robotic arm do not contact with each other, no matter what posture the robotic arm has, or how much the table 1708 is rotated, as long as the table 1708 is maintained parallel to the horizontal plane. Specifically, the robotized bed of this example is configured such that, even when the first movable element 1722 is moved to the lowermost position in a state in which the second movable element 1723 and the third movable element 1724, which are coupled together at their end portions by a horizontally-rotating joint, and the table 1708 are parallel to the horizontal plane, and even when the distal end of the robotic arm is located at the lowermost position, the table 1708 is not level with the other movable elements, nor with the base 1721, and is located at the top. These configurations allow the movable elements of the robotic arm 1701 and the base 1721 to be located and housed under the table 1708, and hence allow effective use of a limited space in the medical settings.

Further, it is recommended that the width of the table 1708 be greater than the width of each of the movable elements of the robotic arm 1701. For example, it is recommended that all the movable elements may be hidden under the table 1708 when viewed from vertically above, in a state in which particular directions of the second movable element 1723 and the third movable element 1724, which are coupled together at their end portions by a horizontally-rotating joint, are parallel to each other when viewed from vertically above. Further, in the present configuration example, it is also recommended that the length of the table 1708, as well, be greater than the length of each of the movable elements of the robotic arm 1701. For example, it is recommended that the base 1721 be hidden under the table 1708 when viewed from vertically above, in a state in which particular directions of the second movable element 1723 and the third movable element 1224, which are coupled together at their end portions by a horizontally-rotating joint, are parallel to each other, and in which the middle portions of the second movable element 1723 and the third movable element 1724 overlap with each other, when viewed from vertically above.

In the examples shown in FIGS. 16 and 17, one (i.e., the second movable element 1723) of the two movable elements (namely, the second movable element 1723 and the third movable element 1724) which are coupled together at their end portions by a horizontally-rotating joint is indirectly coupled to the base 1721 (via the first movable element 1731). However, the second movable element 1723 may be directly connected, for example, to the first joint 1731 traveling vertically straight, or may be more indirectly connected to the base via another horizontally-rotating joint or a vertically-rotating joint. In this case, as well, the advantages of ensuring a larger space and downsizing the robotic arm are achieved, as long as the above-described positional relationship is maintained.

The fourth movable element 1725 is provided at the distal end of the robotic arm 1701. In the present configuration example, the distal end of the robotic arm 1701 is fixed at a lower surface of a middle portion of the table 1708 extending in a particular direction. This configuration allows the robotic arm 1701 to support the table 1708 with great supporting strength, and makes it easier to house the movable elements of the robotic arm 1701, and the base, under the table 1708. Note that the length of the third movable element 1724 may be shortened so that the table 1708 is supported at its one end portion. In this case, as well, the advantages of ensuring a larger space and downsizing the robotic arm are achieved.

The definitions of the "one end portion," "other end portion," "end portion" and "middle portion" as adopted in the above description are the same as, or similar to, those adopted in the first and second configuration examples.

The robotic arm 1701 includes a plurality of actuators (first to sixth actuators 1741-1746 in the present configuration example) associated with the first to sixth joints 1731-1736 to move or rotate the first to fourth movable elements 1722-1725, a plurality of position detectors (first to sixth position detectors 1751-1756 in the present configuration example) built in the respective joints to detect the positions of the respective movable elements, and a controller 1707 (see FIG. 17) which controls the actuation of the respective actuators. The controller 1707 is provided in the base 1721, but may also be an independent external device, for example.

The first to sixth actuators 1741-1746 are servo motors, for example. Similarly to the first and second configuration examples, encoders, resolvers or potentiometers may be used as the position detectors.

It is recommended that the robotic arm 1701 further include first to sixth electromagnetic brakes 1761-1766 associated with the first to sixth joints 1731-1736, respectively. If the robotic arm 1701 does not include any electromagnetic brakes, the posture of the robotic arm 1701 is maintained by actuating the plurality of actuators 1741-1746. If the robotic arm 1701 includes the electromagnetic brakes, the posture of the robotic arm 1701 may be maintained by turning the electromagnetic brakes on even if some of the actuators are turned off.

In the case where the electromagnetic brakes are provided, each of the first to sixth electromagnetic brakes 1761-1766 is configured to turn its brake function on when no drive current is supplied to the associated one of the actuators, and to turn its brake function off when a drive current is supplied to the actuator.

Similarly to the first to third configuration examples, in many cases, a motor functioning as the actuator, an encoder functioning as the position detector, and the brake are integrated together as a unit as shown in FIG. 2. Further, each of the first to sixth actuators 1741-1746 is provided with a deceleration mechanism for power transmission, a coupling, etc.

In the example shown in FIG. 17, the first movable element 1722 is coupled by the horizontally-rotating joint 1732 so as to be located above the second movable element 1723. However, the first movable element 1722 may be coupled by the horizontally-rotating joint 1732 so as to be located under the second movable element 1723. This configuration may compensate for reduction in the height of the base 1721.

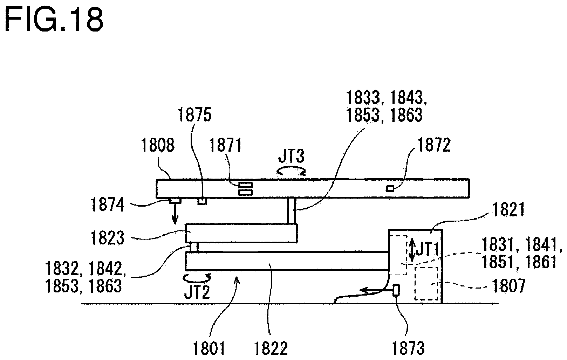

The robotic arm 1701 shown in FIGS. 16 and 17 has 6 degrees of freedom. However, the degrees of freedom of the robotic arm of the present invention do not have to be 6, but may be 5 or less or 7 or more. It is nevertheless recommended that the degrees of freedom of the robotic arm be 3 or more so that the table 1708 can move at least in a straight manner in the room. FIG. 18 shows an example robotized bed having 3 degrees of freedom. In FIG. 18, the robotic arm 1801 is comprised of a base 1821 and two movable elements 1822 and 1823. The base 1821 and one end portion of the first movable element 1822 are coupled together by a first joint 1831 traveling vertically straight, which enables the first movable element 1822 to move in a first axial direction (in a vertical direction). The other end portion of the first movable element 1822 and one end portion of the second movable element 1823 are coupled together by a second joint 1832, which is a horizontally-rotating joint enabling the second movable element 1823 to rotate about a second axis (the vertical direction). The other end portion of the second movable element 1823 serves as the distal end of the robotic arm 1801, and is coupled to a lower surface of a middle portion of the table 1808 by a third joint 1833 which is a horizontally-rotating joint.

The robotized bed having the above configurations makes it possible to move the table 1708•1808, on which the target has been placed, to a target position, such as an inspection position and a treatment position, accurately and quickly, thus achieving significant improvement in the efficiency of the inspection and treatment in medical settings. For example, compared to the configuration in which a table with a caster is used to move the patient, who is a placed target, the table 1708•1808 may be moved more smoothly without shaking the patient too much, and may be prevented from being tangled with a lot of cords of medical equipment and the tubes of medical instruments which run on the floor of the medical room, and may be prevented from being wobbled by stepping over the cords and tubes. This thus improves safety and transfer efficiency.

Examples of the target positions of the robotized bed are the same as, or similar to, those described in the first to third configuration examples, and therefore description thereof will be omitted here.

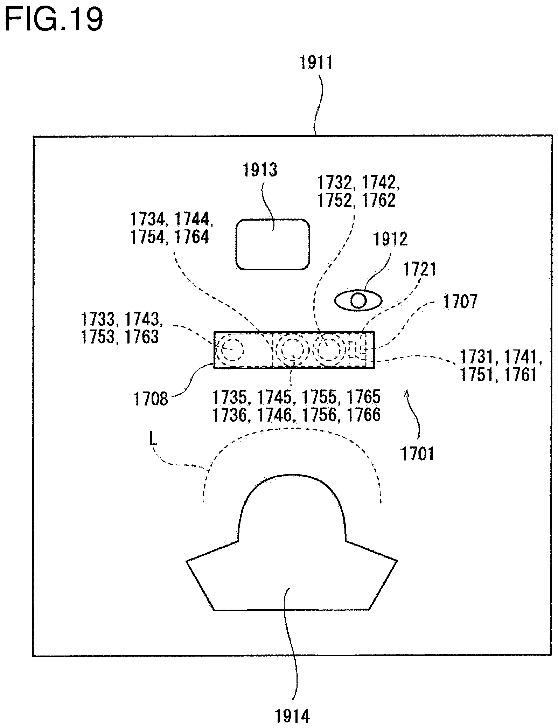

The movements of the table supported by the robotic arm of the present configuration example between the plurality of positions will be described with reference to FIGS. 19-21 by taking, as an example, the robotic arm 1701 having 6 degrees of freedom as shown in FIG. 17.

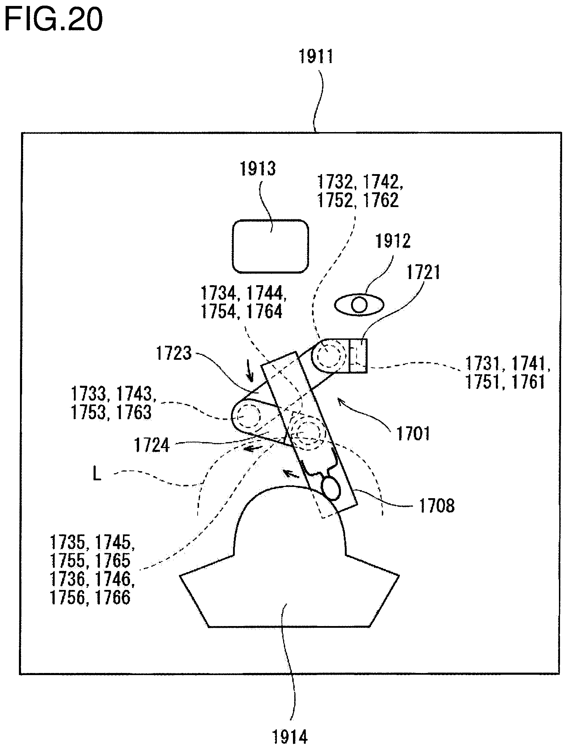

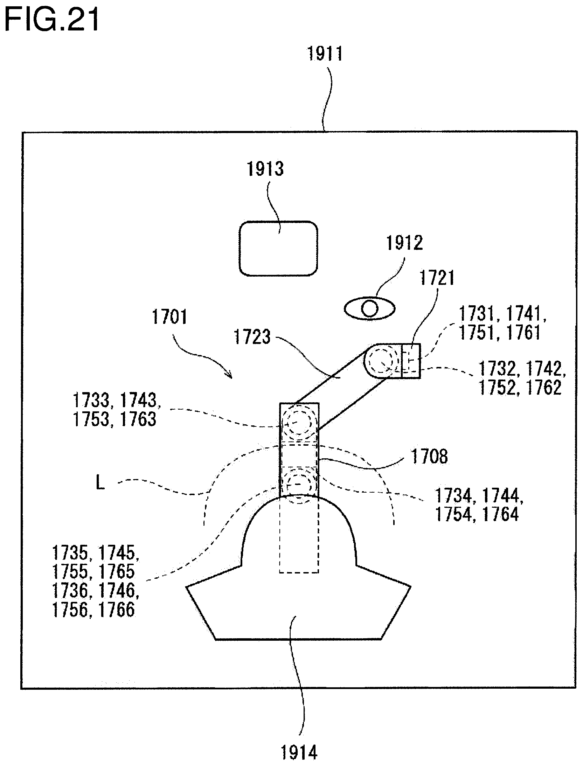

FIG. 19 shows a state in which the table 1708 is located at the placement position in the process of moving a subject, who is a placed target, from the placement position to the inspection position. FIG. 20 shows a state in which the second movable element 1723 and the third movable element 1724 are moved by the control of the controller 1707 as the arrows indicate, and the table 1708 is rotated about the sixth axis as the arrow indicates (in some cases, the first movable element 1722, too, is moved in the vertical direction to have its height adjusted, and the table 1708 is rotated about the fourth axis and/or fifth axis to have its tilt finely adjusted), causing the head of the subject to move toward the inspection device 1914 from an oblique angle. FIG. 21 shows a state in which the table 1708 is inserted in the inspection device 1914, and the subject has arrived at the inspection position. Note that the position of the table 1708 shown in FIG. 19 may also be the treatment position. From the inspection position shown in FIG. 21, the respective movable elements move in reverse direction until the table 1708 returns to the position shown in FIG. 19, where a doctor 1912 can give a treatment based on the result of the inspection which has just been conducted.

The robotic arm 1801 shown in FIG. 18, as well, enables the table 1808 to follow a similar path.

Note that the head of the subject may be placed opposite in the longitudinal direction of the table 1708•1808. In that case, the table 1708•1808 moves to the inspection device 1914, while rotating in the opposite direction to the direction in which the table shown in FIG. 20 rotates. Once the base 1721•1821 is housed in this manner under the table 1708 . 1808, the target may be placed in either direction. If the position of the table 1708•1808 shown in FIG. 19 is the treatment position, the surgeon 1912 may perform surgery from either side of the table 1708•1808, and the surgeon 1912, and assistants, as well, may surround the table during the surgery, which is advantageous. Since the base 1721•1821 does not constitute an obstacle, the doctor 1912 is able to give treatment while seated.

### (Fifth Configuration Example)

A robotized bed according to the present configuration example is characterized by including a slide mechanism, which is provided as an additional member for the table of the robotized bed of each of the first to fourth configuration examples.

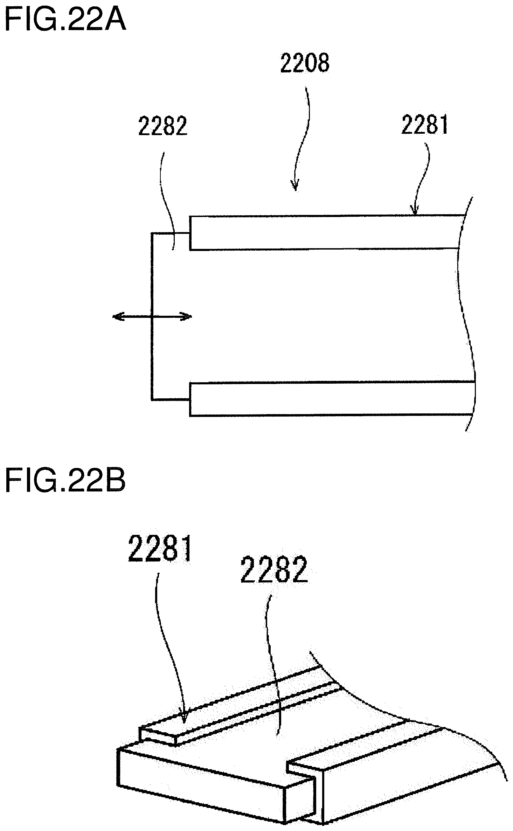

FIGS. 22A and 22B show that the table 2208 is comprised of a body 2281 having rails and a slide plate 2282 fitted in the grooves of the rails. If the table of the robotized bed has this configuration, the slide plate 2282 may be slid by human power to move the placed target farther to an inspection position after the table has been moved to an inspection preparation position by the robotic arm, for example.

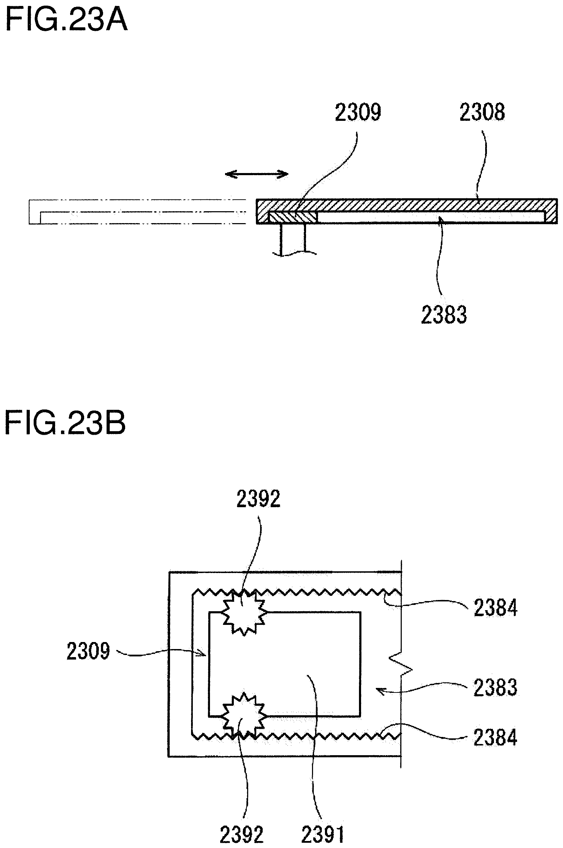

FIGS. 23A and 23B show that the table 2308 has, in its lower surface, a groove 2383 in which a slide mechanism 2309 is fitted. Both sides of the groove 2383 are provided with racks 2384 each having a plurality of teeth. The slide mechanism 2309 includes a body 2391 which is coupled to the distal end of the robotic arm, a pair of pinions 2392 movably supported by the body 2391 and engaged with the respective racks 2384, and an actuator (not shown) which rotates the pinions 2392. If the table 2308 of the robotized bed has this configuration, the table 2308 may be slid by actuating the actuator to move the placed target farther to an inspection position after the table has been moved to an inspection preparation position by the robotic arm, for example. The actuator may be a servo motor, for example.

Note that by providing the slide mechanism, the degree of freedom in each of configuration examples increments by one. In addition, if the slide mechanism is configured to be driven by the actuator, the actuator of the slide mechanism and the plurality of actuators of the robotic arm in the respective configuration examples may be actuated simultaneously so that the movable elements of the robotic arm and the slide mechanism operate simultaneously to transfer the table to the target position efficiently.

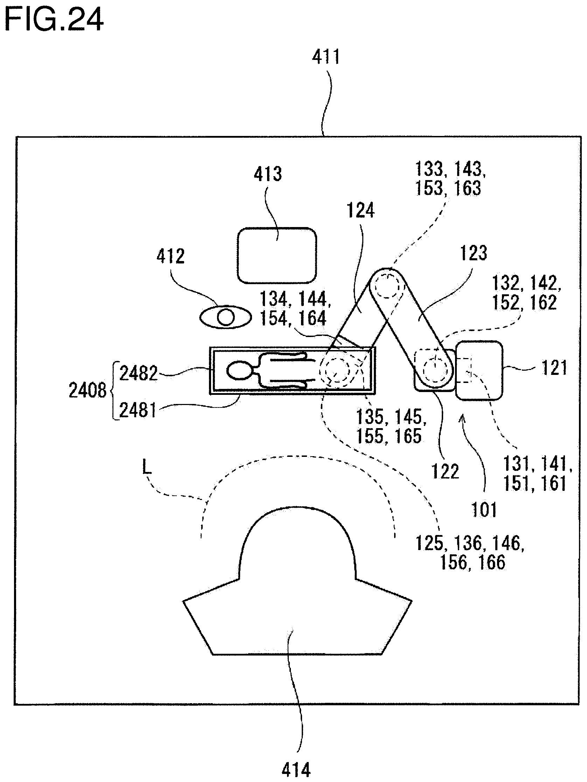

FIGS. 24-26 show example movement of a placed target in a case where a man-powered slide mechanism is adopted as a slide mechanism for the robotized bed of the first configuration example.

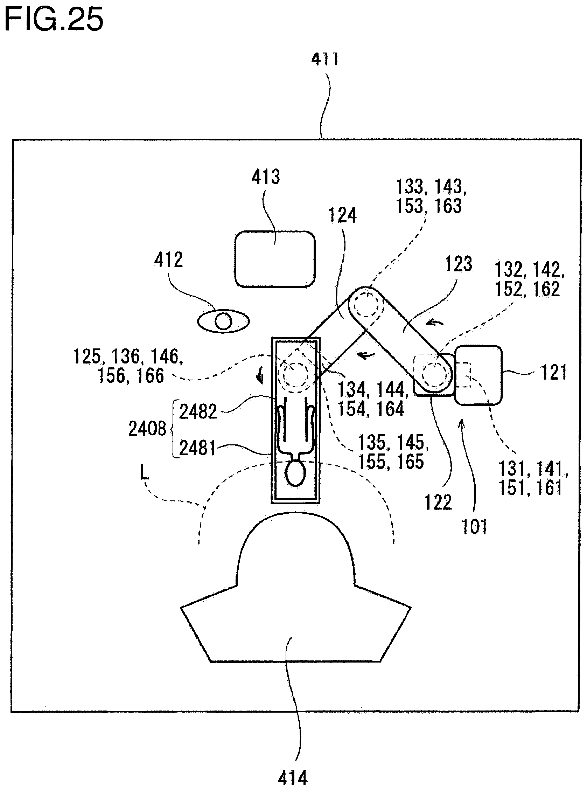

The placement position shown in FIG. 24 where a target is to be placed is the same as the position shown in FIG. 4. The position (i.e., the inspection preparation position) shown in FIG. 25 where the head of the placed target is directed toward the inspection device is the same as the position shown in FIG. 5. In the first configuration example, the table 108 is transferred into the inspection device 414 by simply operating the movable elements of the robotic arm 101, whereas in the present configuration example, the table is transferred into the inspection device 414 by sliding a slide plate by human power.

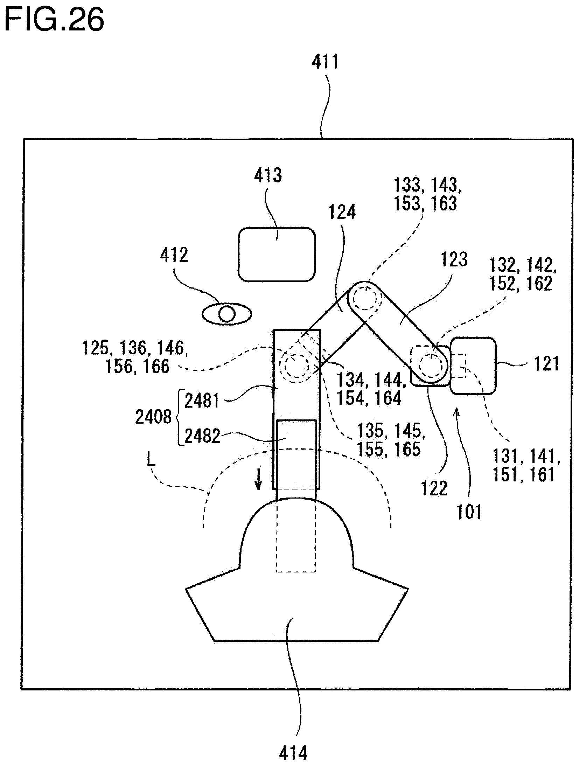

This configuration does not require the robotic arm to go farther than the inspection preparation position, and hence needs only a small range of movement of the robotic arm. Thus, each of the movable elements may be downsized, which is beneficial. Consequently, such a configuration allows effective use of a limited space in medical settings. For example, the second movable element 123 and the third movable element 124 are moved toward the inspection device 414 to make a transition from the state of FIG. 5 to the state of FIG. 6. However, the robotic arm is not moved in making a transition from the state of FIG. 25 to the state of FIG. 26. This reduced movement allows each of the first movable element 123 and the third movable element 124 to have a shorter length.

Now, example movement of a placed target in the case where an actuator-driven slide mechanism is adopted as a slide mechanism for the robotized bed of the third configuration example will be described.

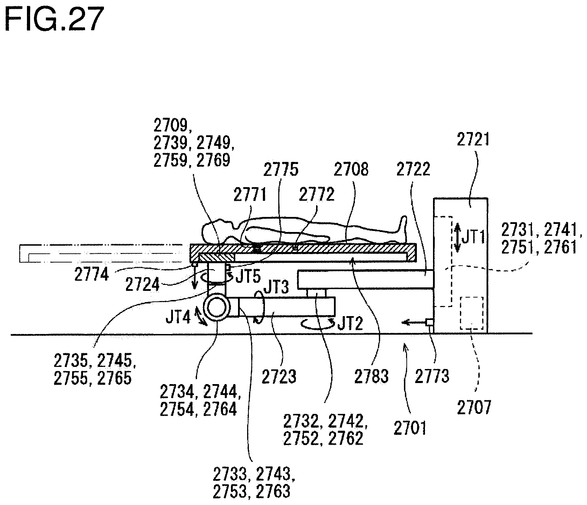

FIG. 27 is a side view of the robotized bed in which the third configuration example is provided with a slide mechanism. The robotic arm 2701 for use in this robotized bed has multiple degrees of freedom (i.e., three or more degrees of freedom), and has a distal end supporting the table 2708 on which a target is placed. The table 2708 and the robotic arm 2701 form the robotized bed.

The robotic arm 2701 includes a base 2721, a plurality of movable elements (first to third movable elements 2722-2724 in the present configuration example), and a plurality of joints (first to fifth joints 2731-2735 in the present configuration example).

The base 2721 and one end portion of the first movable element 2722 are coupled together by the first joint 2731 traveling vertically straight, which enables the first movable element 2722 to move in a first axial direction (in a vertical direction). The other end portion of the first movable element 2722 and one end portion of the second movable element 2723 are coupled together by a horizontally-rotating joint, which enables the second movable element 2723 to rotate about a second axis (the vertical direction). The third to fifth joints 2733-2735 between the second movable element 2723 and the third movable element 2724 are rotating joints which rotate about third to fifth axes, respectively. The third axis corresponds to a direction in which the second movable element 2723 extends. The fourth axis corresponds to a direction orthogonal to the third axis about which the third joint 2733 rotates. The fifth axis corresponds to a direction orthogonal to the fourth axis about which the fourth joint 2734 rotates.

Each of the first movable element 2722 and the second movable element 2723 is a rod-like member extending in a particular direction, with its length appropriately designed according to a required range of movement of the robotic arm 2701. The first movable element 2722 is configured to move up and down, while staying parallel to the horizontal plane, and the second movable element 2723 is configured to rotate about the second axis, while staying parallel to the first movable element 2722. This configuration does not require the second actuator 2742 to compensate for the gravity in the vertical direction, and the motor may thus be reduced in size. This is advantageous in downsizing the robotic arm 2701, and is advantageous in introducing the robotic arm 2701 in the medical settings where only a limited space is available, or in giving a larger space for treatments and surgery.

The third movable element 2724 is provided at the distal end of the robotic arm 2701. In the present configuration example, the distal end of the robotic arm 2701 is coupled to a slide mechanism 2709 of the table 2708.

The robotic arm 2701 includes a plurality of actuators (first to fifth actuators 2741-2745 and an actuator 2749 for slide mechanism in the present configuration example) associated with the first to fifth joints 2731-2735 and the slide mechanism 2709 to move or rotate the first to third movable elements 2722-2724 and the slide mechanism 2709, a plurality of position detectors (first to fifth position detectors 2751-2755 and a position detector 2759 for slide mechanism in the present configuration example) built in the respective joints to detect the positions of the respective movable elements, and a controller 2707 which controls the actuation of the respective actuators. The controller 2707 is provided in the base 2721, but may also be an independent external device, for example.

The first to fifth actuators 2741-2745 and the actuator 2749 for slide mechanism may be servo motors, for example. Similarly to the first and second configuration examples, encoders, resolvers or potentiometers may be used as the position detectors.

It is recommended that the robotic arm 2701 further include first to fifth electromagnetic brakes 2761-2765 and an electromagnetic brake 2769 for slide mechanism which are associated with the first to fifth joints 2731-2735 and the slide mechanism 2709, respectively. If the robotic arm 2701 does not include any electromagnetic brakes, the posture of the robotic arm 2701 is maintained by actuating the plurality of actuators 2741-2745 and the actuator 2749 for slide mechanism. If the robotic arm 2701 includes the electromagnetic brakes, the posture of the robotic arm 2701 may be maintained by turning the electromagnetic brakes on even if some of the actuators are turned off.

In the case where the electromagnetic brakes are provided, each of the first to fifth electromagnetic brakes 2761-2765 is configured to turn its brake function on when no drive current is supplied to the associated one of the actuators, and to turn its brake function off when a drive current is supplied to the actuator.

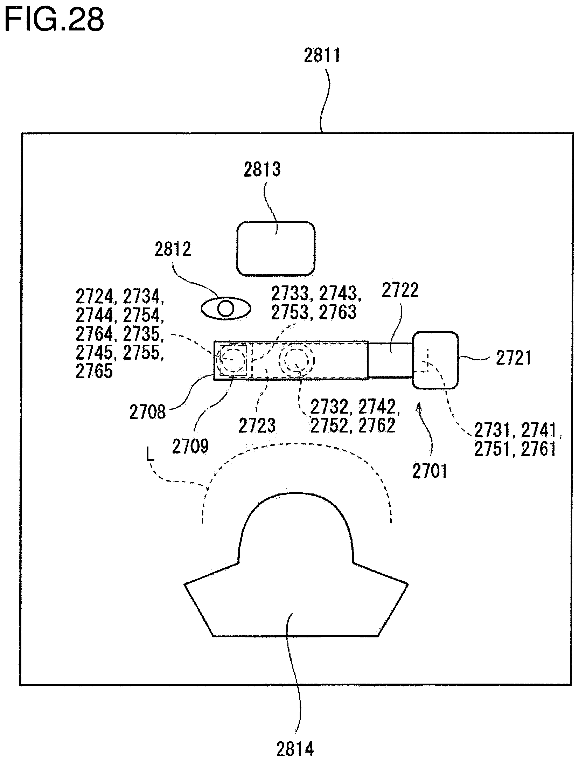

The placement position shown in FIG. 28 where a target is to be placed is the same as the position shown in FIG. 13. However, the robotized bed having a slide mechanism inserts the table 2708 into the inspection device 2814 in the opposite direction. In other words, if the table 1008 shown in FIGS. 13-15 is described as being inserted into the inspection device 1314 from one end of the table 1008, the table 2708 shown in FIG. 28-30 is inserted into the inspection device 2814 from the other end of the table 2708.

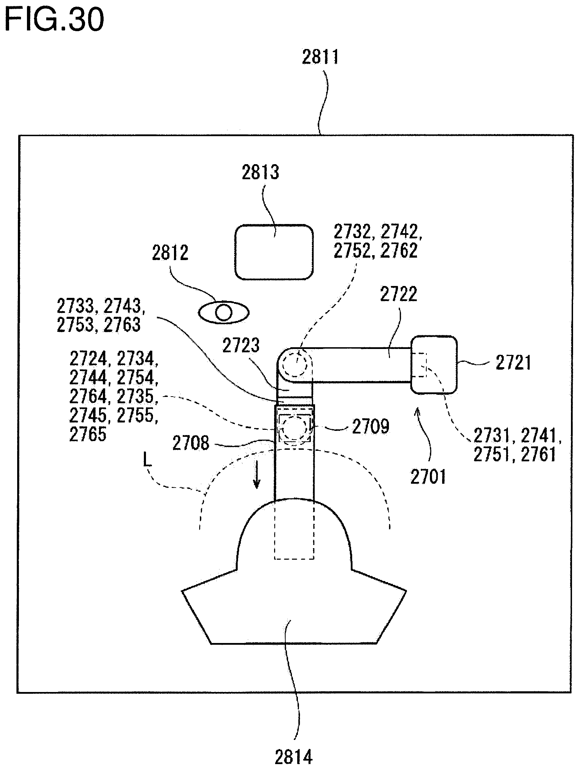

The position (i.e., the inspection position) shown in FIG. 15 where the target is inserted into the inspection device 1314 from his/her head is the same as the position shown in FIG. 30. In the first configuration example, the table 1008 is transferred into the inspection device 1314 by simply operating the movable elements of the robotic arm 1001, whereas in the present configuration example, the table 2708 is once positioned so as to be directed toward the inspection device 2814, and then made to slide into the inspection device 2814 by the actuation of the actuator.

Provision of such a slide mechanism has an advantage of downsizing the robotic arm, and another advantage of changing the orientation of the placed target at the placement position in the third configuration example shown in FIG. 10 (in which the robotic arm 1001 supports the one end portion of the table 1008). As for the latter advantage, in a case, for example, where the placement position is a surgical position where brain or teeth surgery is performed, the surgeon 1312 may have difficulty in performing the surgery if the patient comes back from the inspection device 1314 with his/her head directed toward the base 1021 as shown in FIG. 10, since the base 1021 constitutes an obstacle. On the other hand, if the patient comes back from the inspection device 2814 with his/her head directed away from the base 2721 as shown in FIG. 27, it is easy to perform head surgery. In this case, the base 2721 does not constitute an obstacle, and the doctor 2812 is able to give treatment while seated.

In the two examples described above, the distal end of the robotic arm supports the end portion of the table, but the man-powered slide mechanism may be adopted in the configuration in which the distal end of the robotic arm supports the middle portion of the table. Further, the length of the groove 2783 formed in the table into which the actuator-driven slide mechanism 2709 is fitted may be limited to the length of the middle portion. In that case, the sliding width decreases, but warpage of the table is less likely to occur compared to the case in which the sliding width is great.

Further, in the above examples, the man-powered slide mechanism and the actuator-driven slide mechanism are applied to the first configuration example and the third configuration example, respectively. However, either slide mechanism may be applied to any of the configuration examples.

Adding the slide mechanism to the third configuration example and the fourth configuration example generates a need to change the design of the compact size robotized beds of the third and fourth configuration examples. The fourth configuration example only needs to be configured such that no matter how much the position of the table has been changed by the slide mechanism, the table will not come in contact with the robotic arm irrespective of the angle of rotation of the table, as long as the table is maintained parallel to the horizontal plane. The third configuration example is designed such that in a state in which particular directions of two movable elements, which are coupled together at their end portions by a horizontally-rotating joint, are parallel to each other when viewed from vertically above, the table with the slide mechanism will not contact with the robotic arm, no matter how much (e.g., 360 degrees) the table is rotated parallel to the horizontal plane, from a position closest to the base without moving in the sliding direction. Such design may achieve the advantages obtained by adding the slide mechanism, while maintaining the advantages achieved by the robotized beds in the third and fourth configuration examples.

### [Common Features of Robotic Arms of Respective Configuration Examples]

Additional features applicable to all of the first to fifth configuration examples will be described below.

### (Fixing Member for Tubes/Cords)

If the placed target on the table in each of the configuration examples is a patient, the patient may sometimes be put on a life support system, a drip, or any other equipment necessary for the treatment.

As described above, compared to the configuration in which a table with a caster is moved, the robotized tables of the first to fifth configuration examples may be prevented from being tangled with such tubes (tubes and/or cables) and from being wobbled by stepping over the tubes during the movement of the placed target. To ensure further safety, it is recommended that the robotized bed of the present invention include a fixing member 171 . 371•771•1071•1171•1271•1771•1871•2771 attached to at least one of the table, the base of the robotic arm, or the movable element so as to bundle the tubes extending from the equipment mentioned above. This may prevent a situation in which tubes are tangled during the operation of the robotic arm more reliably. Moreover, doctors or assistants are prevented from tripping over the tubes, which further increases the safety. Tubes for which measures to prevent tangles are necessary are not limited to those connected to the equipment such as a life support system. It is recommended that cords, such as electrical cords for medical equipment and displays, as well, be fixed with the same or similar fixing member. Further, if it is known to which position the table is to be moved, it is recommended that the movement of the robotic arm be roughly predicted to determine how much of the lengths of the tubes/cords should be left unfixed, and where on the tubes/cords the fixing member is to be fitted.

### (Manual Off-Brake Function)

If an electromagnetic brake associated with a horizontally-rotating joint is provided, a switch or a lever for manually turning the brake function off when no drive current is supplied to the actuator may be provided. In the case of the robotic arm 101 shown in FIG. 1, of the first to sixth electromagnetic brakes 161-166, the second, third and sixth electromagnetic brakes 162, 163 and 166 respectively associated with the second, third and sixth joints 132, 133 and 136, which are horizontally-rotating joints, may be configured such that their brake functions are capable of being turned off manually. In the case of the robotic arm 301 shown in FIG. 3, of the first to third electromagnetic brakes 361-363, the second and third electromagnetic brakes 362 and 363 respectively associated with the second and third joints 332 and 333, which are horizontally-rotating joints, may be configured such that their brake functions are capable of being turned off manually. In the case of the robotic arm 701 shown in FIG. 7, of the first to sixth electromagnetic brakes 761-766, the first electromagnetic brake 761 associated with the first joint 731, which is a horizontally-rotating joint, may be configured such that its brake function is capable of being turned off manually. In the case of the robotic arm 1001 shown in FIG. 10, of the first to fifth electromagnetic brakes 1061-1065, the second and fifth electromagnetic brakes 1062 and 1065 respectively associated with the second and fifth joints 1032 and 1035, which are horizontally-rotating joints, may be configured such that their brake functions are capable of being turned off manually. In the case of the robotic arm 1101 shown in FIG. 11, of the first to fifth electromagnetic brakes 1161-1165, the second and fifth electromagnetic brakes 1162 and 1165 respectively associated with the second and fifth joints 1132 and 1135, which are horizontally-rotating joints, may be configured such that their brake functions are capable of being turned off manually. In the robotic arm 1201 shown in FIG. 12, of the first to third electromagnetic brakes 1261-1263, the second and third electromagnetic brakes 1262 and 1263 respectively associated with the second and third joints 1232 and 1233, which are horizontally-rotating joints, may be configured such that their brake functions are capable of being turned off manually. In the case of the robotic arm 1701 shown in FIG. 17, of the first to sixth electromagnetic brakes 1761-1766, the second, third and sixth electromagnetic brakes 1762, 1763 and 1766 respectively associated with the second, third and sixth joints 1732, 1733 and 1736, which are horizontally-rotating joints, may be configured such that their brake functions are capable of being turned off manually. In the case of the robotic arm 1801 shown in FIG. 18, of the first to third electromagnetic brakes 1861-1863, the second and third electromagnetic brakes 1862 and 1863 respectively associated with the second and third joints 1832 and 1833, which are horizontally-rotating joints, may be configured such that their brake functions are capable of being turned off manually. Further, in the case of the robotized bed shown in FIG. 27 which has a motor-driven slide mechanism, the motor which drives the slide mechanism, too, may be provided with an electromagnetic brake, and may be configured such that its brake function is turned off manually.

This configuration allows medical staff to transfer a patient, for example, who is a placed target, to a safe place in the event of a power failure by moving the movable elements of the robotic arm with the brake functions of the movable elements turned off.

Note that the manual off-brake function does not have to be applied to all of the above-listed electromagnetic brakes. Naturally, it may be applied to at least some of the electromagnetic brakes or may be selectively applied to an electromagnetic brake provided at a joint that is movable only parallel to the horizontal plane.

### (Distance Sensor)

It is recommended that the robotic arm of each of the configuration examples be equipped with a distance sensor 173•373•773•1073•1173•1273•1773•1873•2773 which scans the range of movement of the robotized bed. In FIG. 1, the range of movement of the robotic arm 101 forms a sector, of which the radius corresponds to the length from the second axis, about which the second joint 132 rotates, to the distal end of the table 108 when the robotic arm 101 and the table 108 are extended to the maximum. In FIG. 3, the range of movement of the robotic arm 301 forms a sector, of which the radius corresponds to the length from the second axis, about which the second joint 332 rotates, to the distal end of the table 308 when the robotic arm 301 and the table 308 are extended to the maximum. In FIG. 7, the range of movement of the robotic arm 701 forms a sector, of which the radius corresponds to the length from the first axis, about which the first joint 731 rotates, to the distal end of the table 708 when the robotic arm 701 and the table 708 are extended to the maximum. In FIG. 10, the range of movement of the robotic arm 1001 forms a sector, of which the radius corresponds to the length from the second axis, about which the second joint 1032 rotates, to the distal end of the table 1008 when the robotic arm 1001 and the table 1008 are extended to the maximum. In FIG. 11, the range of movement of the robotic arm 1101 forms a sector, of which the radius corresponds to the length from the second axis, about which the second joint 1132 rotates, to the distal end of the table 1108 when the robotic arm 1101 and the table 1108 are extended to the maximum. In FIG. 12, the range of movement of the robotic arm 1201 forms a sector, of which the radius corresponds to the second axis, about which the second joint 1232 rotates, to the distal end of the table 1208 when the robotic arm 1201 and the table 1208 are extended to the maximum. In FIG. 17, the range of movement of the robotic arm 1701 forms a sector, of which the radius corresponds to the length from the second axis, about which the second joint 1732 rotates, to the distal end of the table 1708 when the robotic arm 1701 and the table 1708 are extended to the maximum. In FIG. 18, the range of movement of the robotic arm 1801 forms a sector, of which the radius corresponds to the length from the second axis, about which the second joint 1832 rotates, to the distal end of the table 1808 when the robotic arm 1801 and the table 1808 are extended to the maximum. In FIG. 27, the range of movement of the robotic arm 2701 forms a sector, of which the radius corresponds to the length from the second axis, about which the second joint 2732 rotates, to the distal end of the table 1808 when the table 1808 is extended to the maximum to one side by the robotic arm 2701 and the slide mechanism.

When such a distance sensor 173•373•773•1073•1173•1273•1773•1873•2773 detects a foreign object (a human being or an object) within the range of movement of the robotic arm, the controller 107•307 •707•1007•1107•1207•1707•1807•2707 stops or prohibits the actuation of all the actuators. This configuration reduces risks, such as contact and collision of a human being with the robotic arm or the table, even when the human being, such as medical staff, who is not well acquainted with the robot operation and thus has difficulty in predicting the movement of the robotic arm, is staying close to the robotized bed. Further, other risks, such as contact and collision of the robotic arm with medical equipment, are also avoidable.

It is recommended that the state of the distance sensor be controlled to be active or inactive according to the location of the table in order to prevent the distance sensor from reacting to the doctor or assistant who surrounds the table when, for example, the table arrives at the treatment position. However, it is recommended that a switch that is manually operated to switch the distance sensor between active and inactive states be provided. Alternatively, the state of the distance sensor may also be switched between the active and inactive states by the controller.

### (Height Sensor)

It is recommended that the table or the robotic arm be equipped with a height sensor 174•374•774•1074•1174•1274•1774 •1874•2774 configured to detect the height of the table 108•308•708•1008•1108•1208•1708•1808•2708. In this case, the controller 107•307• 707•1007•1107•1207•1707•1807•2707 determines whether or not the height of the table 108•308•708•1008•1108•1208•1708•1808•2708 detected by the height sensor 174-374-774-1074-1174-1274-1774-1874-2774 is in a predetermined range, before the table 108• 308•708•1008•1108•1208•1708•1808•2708 is moved into the inspection device. If the detected height is not in the predetermined range, the controller 107•307•707•1007•1107• 1207•1707•1807•2707 does not allow the table 108•308•708•1008•1108•1208•1708• 1808-2708 to move into the inspection device. This configuration reduces risks, such as contact and collision of the table or the subject with the inspection device. Although in the above description the inspection position is adopted as an example target position to which the table is transferred, the target position may also be, for example, the measurement position and the imaging position, where the table is inserted in a device related to medical care, i.e., a measurement device and an imaging device, respectively.

### (Warpage Compensation)

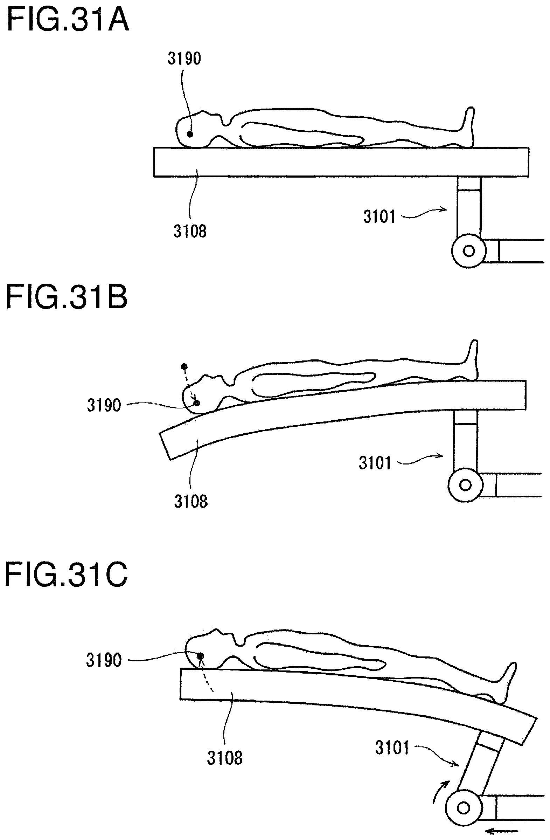

Further, the robotic arm of each of the configuration examples has the function of compensating for warpage of the table or the robotic arm by controlling the robotic arm with the controller according to the degree of warpage of the table or the robotic. FIGS. 31A-31C show how to make correction to the warpage of the table 3108 due to the weight of the placed target, for instance. For example, if one point of the head of the patient, who is a placed target, is determined as a target point to be tracked, the target point 3190 may be stored by, for example, specifying its x, y and z coordinates with respect to the distal end (where the table 3108 is fixed) of the robotic arm 3101 (see FIG. 31A). If the table warps as shown in FIG. 31B, the target point 3190 moves to lower right, for example. The controller of the robotic arm detects this shift of the coordinate values, and controls at least one of the actuators to restore the coordinates of the target point 3190 stored in advance and correct this shift. In the example shown in FIG. 31C, the shift is corrected by moving some movable element of the robotic arm to the left, and rotating the vertically-rotating joint clockwise.

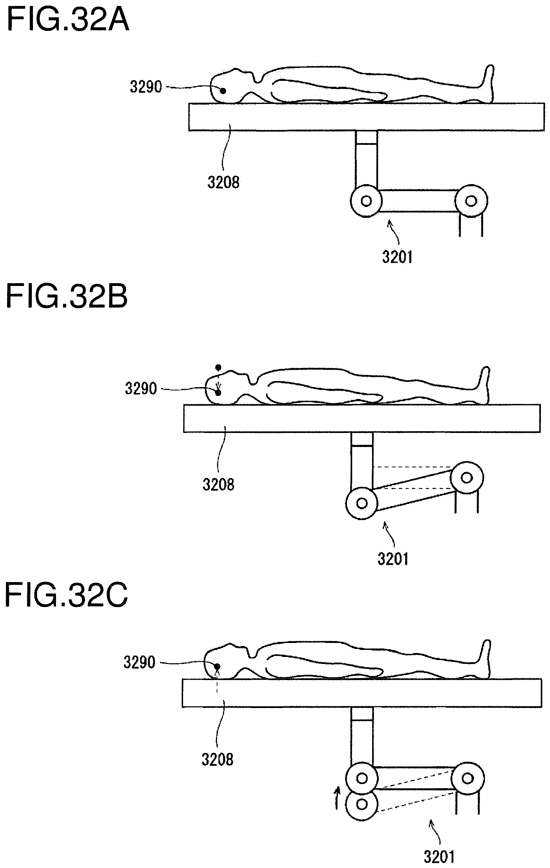

FIGS. 32A-32C show other examples of warpage compensation. For example, similarly to the case shown in FIGS. 31A-31C, if one point of the head of the patient, who is a placed target, is determined as a target point to be tracked, the target point 3290 may be stored by, for example, specifying its x, y, z coordinates with respect to the distal end (where the table 3208 is fixed) of the robotic arm 3201 (see FIG. 32A). If the table warps and the target point 3190 moves down, for example, as shown in FIG. 32B, the controller of the robotic arm detects this shift of the coordinate values, and controls at least one of the actuators to restore the coordinates of the target point 3290 stored in advance and correct this shift. In the example shown in FIG. 32C, the shift is corrected by rotating some vertically-rotating joint of the robotic arm clockwise.

These configurations allow the target point to be always positioned at an accurate point. Thus, a placed target, for example, is transferred to an accurate position. In addition, these configurations reduced risks, such as contact and collision of the table or the placed target with an inspection device, a measurement device, an imaging device, etc.

### (Weight Sensor)

Further, it is recommended that the table or the robotic arm be equipped with a weight sensor 175•375•775•1075•1175•1275•1775•1875•2775 which measures the weight of the placed target. This configuration allows monitoring of the weight of the patient, who is a placed target, all the time. According to this configuration, the patient, who is a placed target, may be monitored in terms of his/her weight. For example, the weight before the start of the surgery may be stored, and the weight reduced by bleeding may be monitored as a reference in determining surgery procedure and changing the surgery strategy. Thus, it is recommended that the table or the robotic arm have a display unit (e.g., a display window or a display) on which numerical values detected by the weight sensor are displayed. Further, it is recommended that this display unit be configured to display a plurality of values recorded (e.g., values recorded before surgery and immediately after the surgery with bleeding) and/or a difference between a stored value and a current value (e.g., a difference between a presurgery value and a current value). To achieve this, it is recommended that a storage device, such as a memory, be provided to store the weight of a placed target in the storage device at some point of time, and that an arithmetic unit, such as a CPU, which calculates a difference between the current weight of the placed target detected by the weight sensor and the weight that has been stored, be provided as well. Further, in order to provide such management for an individual patient, who is a placed target, it is recommended that the storage device be configured to select the patient in association with his/her patient ID, store the weight of the patient at some point of time, calculate the difference between the stored weight and the current weight, and display the difference on the display unit.

### (Temperature Sensor)

Further, it is recommended that the table be equipped with a temperature sensor 172•372•772•1072•1172•1272•1772•1882•2772 which measures the temperature of the placed target. This configuration allows monitoring the temperature of the patient, who is a placed target, all the time. According to this configuration, the patient, who is a placed target, may be monitored in terms of his/her body temperature. For example, the body temperatures before the start of surgery, while waiting for the start of the surgery, during the surgery, and after the surgery may be monitored. It is therefore recommended that the table or the robotic arm have a display unit for displaying thereon the values detected by the temperature sensor.

It is recommended that a temperature increasing device (e.g., a heater) for increasing a surface temperature of the table 108•308•708•1008•1108•1208•1708•1808• 2708 or a temperature decreasing device (e.g., a cooling device) for decreasing the surface temperature of the table 108•308•708•1008•1108•1208•1708•1808•2708 be provided in the event that the body temperature of the patient is too low or too high. This configuration can maintain the patient at a desired body temperature.

In each of FIG. 1, FIG. 3, FIG. 7, FIG. 10, FIG. 11, FIG. 12, FIG. 17, FIG. 18 and FIG. 27, the temperature sensor is arranged on a side surface of the table 108•308•708•1008• 1108•1208•1708•1808•2708. However, the temperature sensor may also be embedded in the table.

Further, another temperature sensor which detects an ambient temperature around the table may be provided to keep the patient at a desired body temperature while he or she is waiting for the start of the surgery and resting after the surgery. The robotic arm may be controlled to move the table to an area where the temperature is low (e.g., to a lower position or close to a cooler) if the ambient temperature is high, or to an area where the temperature is high (e.g., to a higher position or close to a heater) if the ambient temperature is low. Since these automatic movements may be made while the patient is at rest after the surgery or while the patient is waiting for treatment, it is recommended that the table be moved so slowly that the person placed on the table does not sense the movement. However, since the robotic arm should not move automatically during surgery, the temperature sensor may be switched bet active and inactive states according to the area where the table is located. For example, the temperature sensor may be set to be inactive when the table is located at the treatment position.

It is also recommended that each of the temperature sensor and the ambient temperature sensor may be switched manually between the active and inactive states.

### (Object Sensor)

Further, it is recommended that the table be equipped with at least one object sensor for detecting an object around the table, and that the actuation of the actuator driving the robotic arm be stopped or prohibited if the object sensor detects an object while the robotic arm is in motion. Since ensuring safety is very important in utilizing such a robotized bed as described in each of the first to fifth configuration examples in a medical room, it is recommended that the safety of the patient and medical staff be ensured by devices such as this object sensor.

Note that the object sensor may be switched between active and inactive states according to the area where the table is located. For example, the object sensor may be set to be inactive when the table is at the treatment position, or may be set to be active only while the table moves between the placement position where the target is placed and the inspection position. The object sensor may be switched between the active and inactive states by the controller, or may be switched between the active and inactive states with a manual switching member provided at the object sensor.

It is also recommended that each of the temperature sensor and the ambient temperature sensor be manually switched between the active and inactive states.

### (Configuration of Controller)

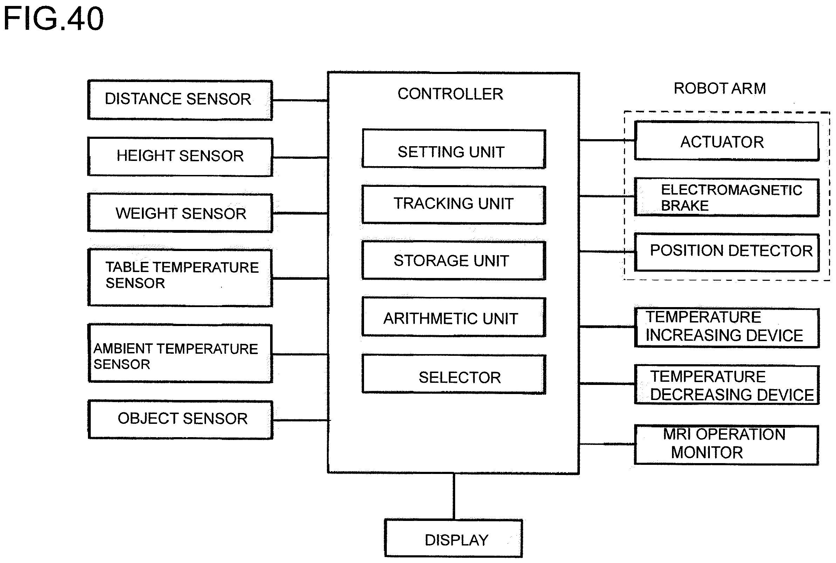

As shown in FIG. 40, the controller 107•307•707•1007•1107•1207•1707•1807• 2707 is connected to the actuators, the electromagnetic brakes and the position detectors of the robotic arm 101, 301, 701, 1001, 1101, 1201, 1701, 1801, 2701. Further, the controller 107•307•707•1007•1107•1207•1707•1807•2707 may be connected to the above described distance sensor 173•373•773•1073•1173•1273•1773•1873•2773, the height sensor 174• 374•774•1074•1174•1274•1774•1874•2774, the weight sensor 175•375•775•1075•1175• 1275•1775•1875•2775, and the temperature sensor 172•372•772•1072•1172•1272•1772• 1882-2772. Moreover, the controller 107•307•707•1007•1107•1207•1707•1807•2707 may include a storage device, and may also include, as a configuration that achieves the above-described warpage compensation, a setting means configured to specify the position of a target point, and a tracking means configured to track the target point.

Further, the controller 107•307•707•1007•1107•1207•1707•1807•2707 may include the above-described storage device and arithmetic unit, or may be connected to the above-described display unit. The display unit may be built in the base of the robotic arm, or may be independent of the robotic arm. Further, if the weights of a plurality of different targets are stored in the storage device of the controller, the controller may include a selector configured to select a particular target to be placed as shown in FIG. 40.

Further, the controller 107•307•707•1007•1107•1207•1707•1807•\2707 may be connected to the above-described temperature increasing device and temperature decreasing device. Moreover, the controller 107•307•707•1007•1107•1207•1707•1807•2707 may be connected to the above-described object sensor.

### [Application to Intraoperative MRI]

The robotized beds described above are expected to achieve significant effects when used in the intraoperative MRI. In the intraoperative MRI for removing brain tumors, the number of times of moving the patient and imaging his/her brain with the MRI apparatus is defined to be 2 to 4, and 3 on average (see "Front-line system for total removal of brain tumor which allows increasing survival rate and ensuring postoperative QOL," Hitachi Medical Corporation, INNERVISION, September (2012)). Thus, there is a high need for moving the patient back and forth between the imaging position, where images are taken by the MRI apparatus, and the treatment position accurately and quickly during surgery.

Described below is a technique for applying the robotized beds (in some cases, the robotized beds with the above-described common additional features) described in the first to fifth configuration examples, to the intraoperative MRI in which images of a specific site of a patient as a placed target are taken by an MRI apparatus, and thereafter the patient is moved to a treatment position (including a surgical position) where surgery is performed immediately.

In the following description, it will be described, with reference to the drawings, how the table 108, 308, 708, 1008, 1108, 1208, 1708, 1808, 2708 is moved between the treatment position and the MRI scanning position by actuating the robotic arm 101, 301, 701, 1001, 1101, 1201, 1701, 1801, 2701.

If the robotized beds of the respective configuration examples are applied to the intraoperative MRI, the apparatuses 414, 1314, 1914 and 2814 placed in the medical room in the description of the movement of the table in the respective configuration examples are MRI apparatuses.

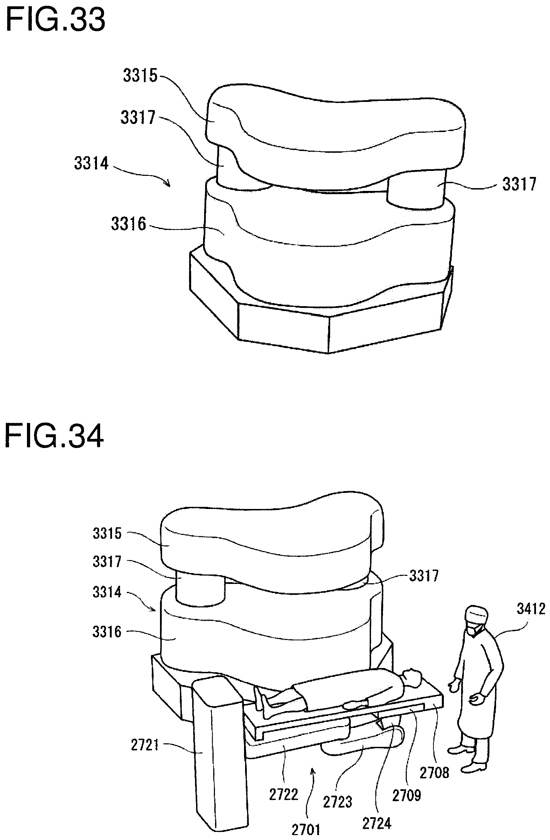

FIG. 33 shows an open MRI apparatus 3314. The open MRI apparatus 3314 is open at the front and lateral sides. Specifically, the open MRI apparatus 3314 includes an upper inspection section (an upper magnet) 3315 and a lower inspection section (a lower magnet) 3316, each of which is in an approximately T-shape with its middle portion protruding forward. Formed between these inspection sections 3315 and 3316 is a space in which the table, where the patient is placed, is to be inserted. The upper inspection section 3315 and the lower inspection section 3316 are coupled together by a pair of support columns 3317 at their respective end portions. The MRI apparatus 3314 may also be a donut-shaped MRI apparatus. However, if the donut-shaped MRI apparatus 3314 is applied to a case (e.g., the case shown in FIG. 14) in which the patient is easily inserted in the MRI apparatus from an oblique angle, the table needs to be positioned in front of the hollow of the donut before being inserted into the hollow, which may make the movement of the robotic arm a little less flexible.

The space defined between the upper inspection section (the upper magnet) 3315 and the lower inspection section (the lower magnet) 3316 is an imaging space. It can be said that the table 108, 308, 708, 1008, 1108, 1208, 1708, 1808, 2708 is in the MRI scanning position when at least part of the table 108, 308, 708, 1008, 1108, 1208, 1708, 1808, 2708 overlaps with this imaging space. The position of the table 108, 308, 708, 1008, 1108, 1208, 1708, 1808, 2708 in the imaging space is not always the same, since it differs depending on a site to be imaged of the patient and the height and size of the patient.

FIG. 4 shows a state in which the table 108 is located at the placement position in the process of moving a patient, who is a placed target, from the placement position to the MRI scanning position, using the robotized bed of the first configuration example. FIG. 5 shows a state in which the second movable element 123 and the third movable element 124 are moved by the control of the controller 107 as the arrows indicate, and the table 108 is rotated about the sixth axis as the arrow indicates (and in some cases, the first movable element 122, too, is moved in the vertical direction to have its height adjusted, and the table is rotated about the fourth axis and/or the fifth axis to have its tilt finely adjusted), causing the head of the patient to be directed toward the MRI apparatus 414 (i.e., the state in which the patient is located at an MRI scanning preparation position). FIG. 6 shows a state in which the table 108 is inserted in the MRI apparatus 414, and the table 108 has arrived at the MRI scanning position. If the table 108 needs to be moved to the treatment position after the MRI apparatus 414 takes images so that the surgeon 412 can perform surgery on the patient, the respective movable elements and the table 108 are moved in reverse direction until the table 108 returns from the MRI scanning position shown in FIG. 6 to the position shown in FIG. 4, where the surgeon 412 can immediately start the surgery while looking at the MRI images.

FIG. 13 shows a state in which the table 1008 is located at the placement position in the process of moving a patient, who is a placed target, from the placement position to the MRI scanning position, using the robotized bed of the third configuration example. FIG. 14 shows a state in which the second movable element 1023 and the table 1008 are moved by the control of the controller 1007 as the arrows indicate (in some cases, the first movable element, too, is moved in the vertical direction to have its height adjusted, and the table 1008 is rotated about the third axis and/or the fourth axis to have its tile finely adjusted), causing the head of the patient to move toward the MRI apparatus 1314 from an oblique angle. FIG. 15 shows a state in which the table 1008 is inserted in the MRI apparatus 1314, and the patient has arrived at the inspection position. If the table 1008 needs to be moved to the treatment position after the MRI apparatus 1314 takes images so that the surgeon 1312 can perform surgery on the patient, the respective movable elements and the table 1008 are moved in reverse direction until the table 1008 returns from the MRI scanning position shown in FIG. 15 to the position shown in FIG. 13, where the surgeon 1312 can immediately start the surgery while looking at the MRI images.

FIG. 19 shows a state in which the table 1708 is located at the placement position in the process of moving a patient, who is a placed target, from the placement position to the MRI scanning position, using the robotized bed of the fourth configuration example. FIG. 20 shows a state in which the second movable element 1723 and the third movable element 1724 are moved by the control of the controller 1707 as the arrows indicate, and the table 1708 is rotated about the sixth axis as the arrow indicates (in some cases, the first movable element 1722, too, is moved in the vertical direction to have its height adjusted, and the table 1708 is rotated about the fourth axis and/or fifth axis to have its tilt finely adjusted), causing the head of the patient to move toward the MRI apparatus 1914 from an oblique angle. FIG. 21 shows a state in which the table 1708 is inserted in the MRI apparatus 1914, and the table 1708 has arrived at the MRI scanning position. If the table 1708 needs to be moved to the treatment position after the MRI apparatus 1914 takes images so that the surgeon 1912 can perform surgery on the patient, the respective movable elements and the table 1708 are moved in reverse direction until the table 1708 returns from the MRI scanning position shown in FIG. 21 to the position shown in FIG. 19, where the surgeon 1912 can immediately start the surgery while looking at the MRI images.

FIGS. 24-26 show a fifth configuration example applied to the intraoperative MRI. In the present fifth configuration example, a man-powered slide mechanism is added to the robotized bed of the first configuration example.

The placement position shown in FIG. 24 where a target is to be placed is the same as the position shown in FIG. 4. The position (i.e., the MRI scanning preparation position) shown in FIG. 25 where the head of the placed target is directed toward the MRI apparatus 414 is the same as the position shown in FIG. 5. In the case of the robotized bed of the first configuration example, the table 108 is transferred into the MRI apparatus 414 by simply operating the movable elements of the robotic arm 101, whereas in the case of the robotized bed of the fifth configuration example, the table 108 is moved into the MRI apparatus 414 by sliding, at the MRI scanning preparation position, the slide plate 2481 by human power.

FIGS. 28-30 show the fifth configuration example applied to the intraoperative MRI. In the present fifth configuration example, an actuator-driven slide mechanism is added to the robotized bed of the third configuration example.

The placement position shown in FIG. 28 where a patient is to be placed is the same as the position shown in FIG. 13. However, the robotized bed having a slide mechanism inserts the table 2708 into the MRI apparatus 2814 in the opposite rotational direction. In other words, if the table 1008 shown in FIGS. 13-15 is regarded as being inserted into the inspection device 1314 from one end of the table 1008, the table 2708 shown in FIG. 28-30 is inserted into the MRI apparatus 2814 from the other end of the table 2708.

The position (i.e., the MRI scanning position) shown in FIG. 15 where the target is inserted into the MRI apparatus 1314 from his/her head is the same as the position shown in FIG. 30. In the case of the robotized bed of the third configuration example, the table 1008 is transferred into the MRI apparatus 1314 by simply operating the movable elements of the robotic arm 1001, whereas in the case of the robotized bed of the fifth configuration example, the table 2708 is positioned so as to be directed toward the MRI apparatus 2814, and then made to slide into the MRI apparatus 2814 by the actuation of the actuator.

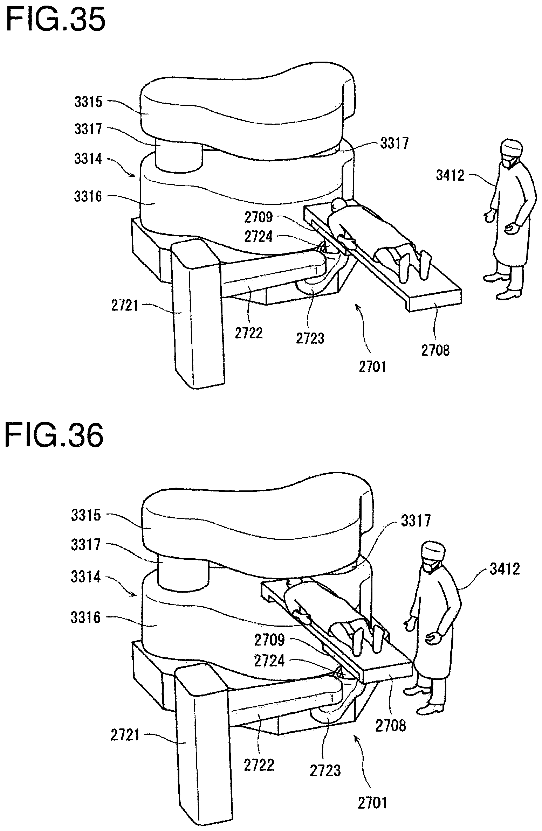

FIGS. 34-36 show perspective views of the movements of the robotized bed according to the fifth configuration example applied to the intraoperative MRI. In the present fifth configuration example, an actuator-driven slide mechanism is added to the robotized bed of the third configuration example. The position shown in FIG. 34 is the placement position, where a patient is placed on the table, and the surgical position. The second movable element 2723 is rotated in the horizontal direction, and the table 2708 simultaneously rotates about the fifth axis, causing the table 2708 to move to the MRI scanning preparation position shown in FIG. 35. Then, the table 2708 is made to slide by the actuation of the actuator to a position where the table 2708 overlaps with the imaging space of the MRI apparatus. The transfer of the table 2708 to the MRI scanning position is completed there.

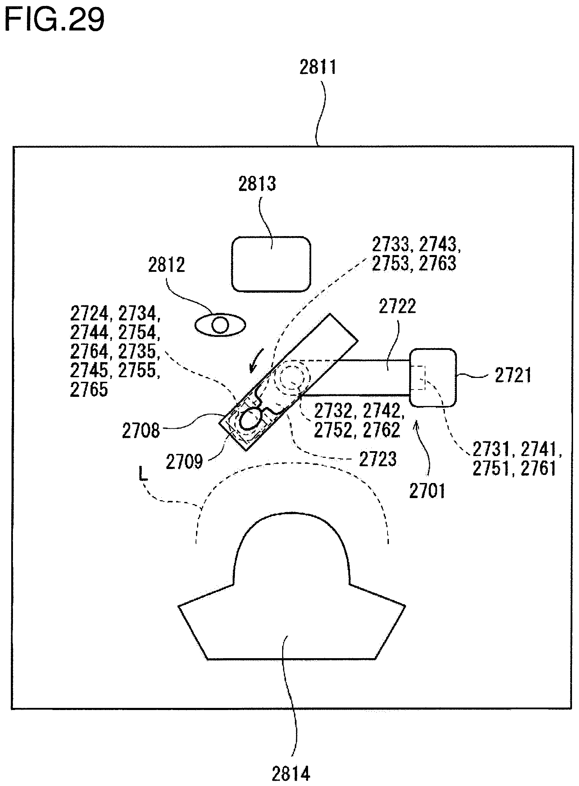

The second movable element 2723 at the MRI scanning preparation position shown in FIG. 35 differs in its orientation from the second movable element 2723 at the MRI scanning preparation position during the transition from the position in FIG. 29 to the position in FIG. 30 (during the transition from FIG. 29 to FIG. 30, the second movable element 2723 faces the MRI apparatus 2814 at right angles, whereas in FIG. 35 the second movable element 2723 is oblique with respect to the MRI apparatus 2814). However, the movement of the robotic arm differs depending on where to arrange the MRI apparatus and the dimensions of the respective movable elements.

An advantage in using the robotized bed of the fifth configuration example, which has a slide mechanism, is that it is possible to downsize the robotic arm. An advantage in using the robotized bed of the third configuration example shown in FIG. 10 (in which the robotic arm 1001 supports one end portion of the table 1008) is that it is possible to change which side the head of the patient is directed to at the treatment position. As for the latter advantage, in a case, for example, where the intraoperative MRI is used to carry out surgery for removing a brain tumor or teeth surgery, the surgeon 1312 may have difficulty in performing the surgery if the patient comes back from the MRI apparatus 1314 with his/her head directed toward the base 1021 as shown in FIG. 10, since the base 1021 constitutes an obstacle. On the other hand, if the patient comes back from the MRI scanning position with his/her head directed away from the base 2721 as shown in FIG. 27, it is easy for the surgeon 1312 to perform head surgery. In this case, the base 2721 does not constitute an obstacle around the head of the patient during the surgery, and the surgeon 2812 is able to give treatment while seated.

Note that the MRI scanning preparation positions shown in FIG. 5 and FIG. 25 are positions where the table 108·2408 does not overlap with the imaging space of the MRI apparatus, and are located close to the imaging space (within a predetermined distance from the imaging space). The movement of the table 108·2408 may be stopped for a while at this MRI scanning preparation position, where an assistant, for example, may prepare for the MRI (e.g., check if there is no metallic object, and correct the position and posture of the patient), and thereafter the table 108·2408 may be transferred to the MRI apparatus. Naturally, the table may just pass through the MRI scanning preparation position without stopping there, and smoothly move to the MRI scanning position. Further, the MRI scanning preparation position does not have to be a place where the head of the patient faces the MRI apparatus directly. For example, the position of the table 1008 shown in FIG. 14, in which the table 1008 is located close to the imaging space, may be the MRI scanning preparation position.

Further, in the above description, an example has been described in which a patient is transferred from the placement position to the MRI scanning position and then the patient is returned to the same placement position which now serves as the treatment position. However, the treatment position may be different from the placement position where the patient has been placed on the table.

The treatment position in the intraoperative MRI is located at a position where the table is not close to the imaging space, that is, a position located at least at a predetermined distance from the imaging space. In the above examples, a surgical instrument table 413 . 1313·1913 - 2813, on which surgical instruments to be used by the surgeon 412·1312·1912· 2812 are placed, is disposed near the treatment position. If these surgical instruments are placed close to the MRI apparatus, the surgical instruments may be affected (e.g., may float) by the permanent magnet of the MRI apparatus, and may hurt the patient and those who handle the surgical instruments. It is therefore recommended that the treatment position be sufficiently away from the MRI apparatus, preferably farther away from the 5 Gauss line L.

It is also recommended that the base 121, 321, 721, 1021, 1121, 1221, 1721, 1821, 2721 of the robotic arm be located outside the 5 Gauss line L. The base 121, 321, 721, 1021, 1121, 1221, 1721, 1821, 2721 of the robotic arm is provided with a big motor. If this motor is located close to the MRI apparatus, the magnetic field generated at the imaging space of the MRI apparatus is distorted, which leads to a deterioration of the MRI images.

Using the robotic arms of the first to fifth configuration examples allows the table 108, 308, 708, 1008, 1108, 1208, 1708, 1808, 2708 to be apart from the MRI apparatus 414· 1314·1914·2814 by the length of the robotic arm 101, 301, 701, 1001, 1101, 1201, 1701, 1801, 2701. This makes it possible to keep the treatment position apart from the MRI apparatus 414·1314·1914·2814 by a distance twice as long as the robotic arm 101, 301, 701, 1001, 1101, 1201, 1701, 1801, 2701 at maximum. In other words, the treatment position may be easily set outside the 5 Gauss line L by using the robotic arm 101, 301, 701, 1001, 1101, 1201, 1701, 1801, 2701. As a result, the surgeon 412·1312·1912·2812 may be less affected, and hence less burdened, by the magnetic field. In addition, the surgeon 12 may stand anywhere he/she likes.

As can be seen from the foregoing description, application of the robotized beds described in the first to fifth configuration examples to the intraoperative MRI allows the patient placed on the table to be moved between the treatment position and the MRI scanning position quickly and accurately by the operation of the robotic arm. This may contribute to enhancing the superior effect of improving the performance of surgery. According to the aforementioned document, "Front-line system for total removal of brain tumor which allows survival rate increase and ensures postoperative QOL," Hitachi Medical Corporation, INNERVISION, September (2012), compared to the conventional brain tumor removal surgery in which the MRI and surgery have been performed in different rooms, application of the intraoperative MRI in which imaging and surgery are performed in the same room (and further application of information-guided surgery) achieves five-year survival rates of 78% in grade 3 and 19% in grade 4, which are about three times the average conventional five-year survival rates of about 25% in grade 3 and about 7% in grade 4 of the surgery performed in different rooms. Application of the robotized beds of the first to fifth configuration examples to the intraoperative MRI allows the table on which the patient is placed to be transferred quickly and accurately as described so far, and allows the MRI scanning and the brain tumor removal surgery to be performed efficiently. Also, these robotized beds are highly expected to contribute to further improving the survival rate. In particular, as explained earlier, in the brain tumor removal surgery, the MRI scanning and the brain tumor removal surgery are not performed only once, but are repeated several times. Thus, there are high expectations for the quick and accurate transfer of the patient between the treatment position and the MRI scanning position.

In applying the robotized beds of the first to fifth configuration examples to the intraoperative MRI, it is recommended that the supply of the drive current to the plurality of actuators mounted on the robotic arm 101, 301, 701, 1001, 1101, 1201, 1701, 1801, 2701 be stopped and the brake functions of the plurality of electromagnetic brakes associated with the actuators be turned on by the control of the controller 107·307·707·1007·1107·1207·1707· 1807·2707, during a period after the table 108, 308, 708, 1008, 1108, 1208, 1708, 1808, 2708 has arrived at the MRI scanning position and before images of the target placed on the table starts to be taken. This is recommended to reduce the deterioration of the MRI images due to the magnetic field generated while the actuators are actuated, for the MRI apparatus takes images by utilizing the static magnetic field. This control may be automatically carried out when the controller detects that the table has arrived at the MRI scanning position and stayed there for a predetermined period of time, or may be carried out in accordance with a manually entered instruction. It is recommended, however, that the start of MRI scanning (e.g., at a time when the main power of the MRI apparatus is turned on, or the MRI apparatus is turned into an active state) trigger the checkout to determine whether the actuators of the robotic arm are actuated or not. If the actuators are actuated, the actuators are turned off intentionally to have the brake functions turned on. It is therefore recommended that the controller 107· 307·707·1007·1107·1207·1707·1807·2707 have an MRI operation monitor to monitor, for example, whether the main power of the MRI apparatus is turned on or whether the MRI apparatus is turned into an active state.

The robotic arm of the fifth configuration example may be provided with a man-powered slide mechanism. Thus, the supply of the drive current to the plurality of actuators mounted on the robotic arm 101, 301, 701, 1001, 1101, 1201, 1701, 1801, 2701 may be stopped and the brake functions of the plurality of electromagnetic brakes associated with the actuators may be turned on by the control of the controller 107·307·707·1007·1107·1207· 1707·1807·2707 at a time when the table 108, 308, 708, 1008, 1108, 1208, 1708, 1808, 2708 arrives at the MRI scanning preparation position. After the actuators are turned off and the brake functions of the electromagnetic brakes are turned on, the slide plate is made to slide to move the patient to the MRI scanning position.

The table may be moved between the surgical position and the MRI scanning position by actuating the robotic arm 101, 301, 701, 1001, 1101, 1201, 1701, 1801, 2701 through a teaching pendant. However, if the surgical position and the MRI scanning position are stored in advance in the controller 107·307·707·1007·1107·1207·1707·1807· 2707, the table 108, 308, 708, 1008, 1108, 1208, 1708, 1808, 2708 may move between the surgical position and the MRI scanning position more quickly and smoothly.

If the robotic arm automatically moves the table between the surgical position and the MRI scanning position, it depends on the accuracy of positioning of the robotic arm whether or not the surgical field may be returned to the same place with reliability after the MRI scanning. Further, an advantage in using the robotic arm is that a large surgical field can be obtained during surgery by changing the position and posture of the patient by operating the robotic arm during the surgery.

### [Application to Other Treatments]

The robotized beds described in the first to fifth configuration examples (in some cases, the robotized beds with the above-described common additional features) may be applied not only to the intraoperative MRI, but also to other treatments as well.

For example, the apparatus 414 shown in FIGS. 4-6 and FIGS. 24-26, the apparatus 1314 shown in FIGS. 13-15, the apparatus 1914 shown in FIGS. 19-21, and the apparatus 2814 shown in FIGS. 28-30 which were referred to when the movement of the table was described in the respective configuration examples may be X-ray machines. After a patient is placed on the table 108, 308, 708, 1008, 1108, 1208, 1708, 1808, 2708, the table is moved to the imaging position to x-ray the teeth of the patient, and successively moved to the treatment position to give teeth treatment.

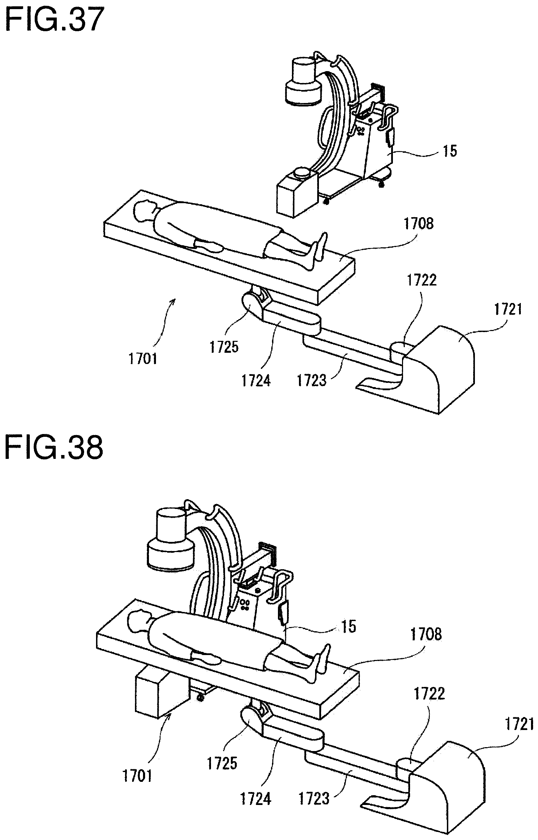

Another example is that the apparatus 414 shown in FIGS. 4-6 and FIGS. 24-26, the apparatus 1314 shown in FIGS. 13-15, the apparatus 1914 shown in FIGS. 19-21 and the apparatus 2814 shown in FIGS. 28-30 which were referred to when the movement of the table was described in the respective configuration examples may be angiographic apparatuses. After a patient is placed on the table 108, 308, 708, 1008, 1108, 1208, 1708, 1808, 2708, the table is moved to the imaging position to take images of a target site by X-ray fluoroscopy using the angiographic apparatus 15, and thereafter moved to the treatment position to give catheter treatment or any other treatment. In this case, the angiographic apparatus 15 is fixed and the robotic arm 1701 is operated to insert the table 1708 into a C-shaped arm. However, as shown in the appearance views of FIGS. 37 and 38, the table 1708 may be inserted into the C-shaped arm of the angiographic apparatus 15 by moving the table 1708 to the imaging position first, and then moving the angiographic apparatus 15 toward the table 1708.

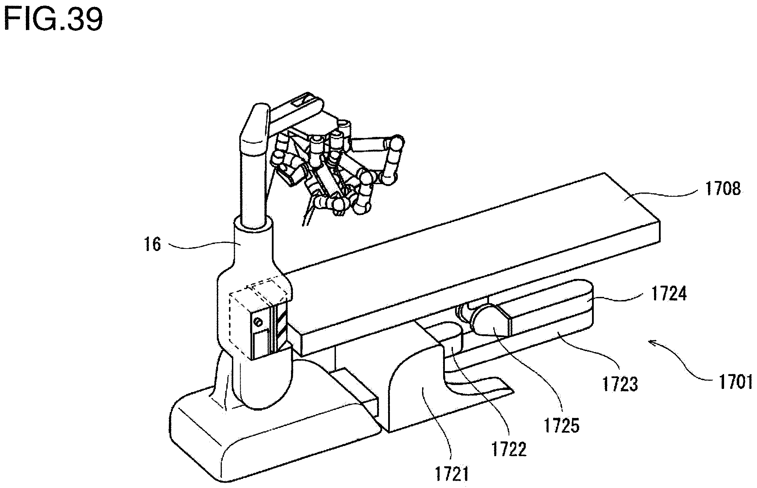

Still another example is that a surgical robot is arranged at the treatment position shown in FIGS. 4-6, FIGS. 13-15, FIGS. 19-21, FIGS. 24-26 and FIGS. 28-30 which were referred to when the movement of the table was described in the respective configuration examples. At the treatment preparation position, a cannula, for example, is inserted in a patient to make the patient ready for laparoscopic surgery, and thereafter the patient is moved to the treatment position where the surgical robot performs the laparoscopic surgery with its remotely-controlled manipulator. FIG. 39 shows a state in which the table 1708 of the robotized bed of the fourth configuration example has been moved to the treatment position where a surgical robot is arranged.

In these cases, as well, the above-mentioned common features may be added. For example, if the robotized beds of the first to fifth configuration examples are used to move the table to the position where images are taken by the angiographic apparatus 15, the above-described height sensor 174·374•774•1074•1174•1274•1774•1874•2774 may be provided. If the height of the table 108, 308, 708, 1008, 1108, 1208, 1708, 1808, 2708 detected by the height sensor is not within the opening area of the C-shaped arm, the movement of the angiographic apparatus or the movement of the table by the robotic arm may be stopped.

Examples in which the robotized beds of the first to fifth configuration examples are applied to various scenes in the medical settings have been described above. However, the present invention may be modified in various manners without departing from the scope of the present invention. For example, the foregoing description has been made on the premise that the base 121, 321, 721, 1021, 1121, 1221, 1721, 1821, 2721 of the robotic arm is fixed. However, depending on the layout of the medical room, the base may be installed on a rotating floor, and may be moved in accordance with the rotation of the floor. Further, a medical room may be provided with a rail via which the base can move. In these configurations, too, in which the base itself is movable, the table can move to the above respective positions by combining the movement of the table with the control of the robotic arm.

Note that the terms "bed" and "table" used in the above description are synonyms, and different terms may have been used to clarify the portion being described.

### DESCRIPTION OF REFERENCE CHARACTERS

101, 301, 701, 1001, 1101, 1201, 1701, 1801, 2701, 3101, 3201: Robotic Arm
414, 1314, 1914, 2814, 3314: MRI Apparatus
121, 321, 721, 1021, 1121, 1221, 1721, 1821, 2721: Base
122-125, 322-323, 722-724, 1022-1024, 1122-1124, 1222-1223, 1722-1725, 1822-1823, 2722-2724: Movable Element
131-136, 331-333, 731-736, 1031-1035, 1131-1135, 1231-1233, 1731-1736, 1831-1833, 2731-2735: Joint
141-146, 341-343, 741-746, 1041-1045, 1141-1145, 1241-1243, 1741-1746, 1841-1843, 2741-2745: Actuator
151-156, 351-353, 751-756, 1051-1055, 1151-1155, 1251-1253, 1751-1756, 1851-1853, 2751-2755: Position Detector
161-166, 361-363, 761-766, 1061-1065, 1161-1165, 1261-1263, 1761-1766, 1861-1863, 2761-2765: Electromagnetic Brake
171, 371, 771, 1071, 1171, 1271, 1771, 1871, 2771: Fixing Member
172, 372, 772, 1072, 1172, 1272, 1772, 1872, 2772: Temperature Sensor
173, 373, 773, 1073, 1173, 1273, 1773, 1873, 2773: Distance Sensor
174, 374, 774, 1074, 1174, 1274, 1774, 1874, 2774: Height Sensor
175, 375, 775, 1075, 1175, 1275, 1775, 1875, 2775: Weight Sensor
107, 307, 707, 1007, 1107, 1207, 1707, 1807, 2707: Controller
108, 308, 708, 1008, 1108, 1208, 1708, 1808, 2308, 2408, 2708, 3108, 3208: Table

## Claims

1. A robotized bed comprising:
a robotic arm (101) which includes two movable elements, a first moveable (122) element and a second moveable element (123), coupled together at one of their end portions by a horizontally-rotating joint (132); and
a table (108) for placing a patient rotatably supported by a distal end of the robotic arm, wherein
the robotized bed is configured so that the table and the robotic arm do not come in contact with each other, when the robotic arm rotates the table while the table is maintained parallel to a horizontal plane in a state which the robotic arm takes an arbitrary posture, and
the robotized bed is configured to take a posture in which the robotic arm is hidden in its entirety under the table when viewed from vertically above.

2. The robotized bed of claim 1, wherein
the robotic arm further includes a base (121) that is lower in height than a lower surface of the table, when the distal end of the robotic arm is located at a lowermost position and the table is in a posture parallel to the horizontal plane.

3. The robotized bed of claim 2, wherein
the first movable element and the base are coupled together by a joint (131) traveling vertically straight.

4. The robotized bed of any one of claims 1-3, wherein
the distal end of the robotic arm supports one end portion or a middle portion of the table.

5. The robotized bed of any one of claims 1-4, wherein
the table has a table slide mechanism (2309).

6. The robotized bed of claim 5, wherein
the table slide mechanism is controlled and configured such that the table is slid in a direction parallel to the horizontal plane by actuating an actuator or is slid by human power.

7. The robotized bed of any one of claims 1-6, comprising:
a distance sensor (173) which scans a range of movement of the table or the robotic arm, wherein
when the distance sensor detects an object in the range of movement, movement of the robotic arm is stopped.

8. The robotized bed of any one of claims 1-7, wherein
the robotized bed is configured to track a target point, and if an actual position is shifted from the target point due to warpage of the table, a posture of the robotic arm is changed to make a correction to the shift and bring the actual position to the target point.

9. The robotized bed of any one of claims 1-8, comprising:
electromagnetic brakes (161-166) of which a break function is turned on when no drive current is supplied to any of an actuator which operates the two movable elements rotating about the rotating joint which couples the two movable elements and an actuator which operates the table, and of which the break function is turned off when the drive current is supplied to the actuators, wherein
at least one of the electromagnetic brakes is configured so that the break function is capable of being turned off manually when the drive current is not supplied.

10. The robotized bed of any one of claims 1-9, wherein
the robotic arm further comprises a third movable element (124) coupled to the second movable element by a second horizontally-rotating joint (133).

11. The robotized bed of claim 10, wherein
the robotic arm further comprises a fourth movable element (125) coupled to a rotating joint (135) which is configured to rotate in a direction orthogonal to a longitudinal direction of the third movable element.

12. The robotized bed of claims 1-11, wherein
the robotic arm is configured to move the table to a plurality of predetermined positions including a treatment position where a doctor treats the patient and an imaging position where an imaging device images the patient.

13. The robotized bed of claim 12, wherein
the imaging device is an MRI apparatus (414) or an angiographic apparatus (15).

## Patentansprüche

1. Robotisiertes Bett, umfassend:
einen Roboterarm (101), der zwei bewegliche Elemente umfasst, ein erstes bewegliches Element (122) und ein zweites bewegliches Element (123), die an einem ihrer Endabschnitte durch ein horizontal drehbares Gelenk (132) miteinander gekoppelt sind; und
einen Tisch (108) zum Platzieren eines Patienten, der drehbar von einem distalen Ende des Roboterarms getragen wird, wobei
das robotisierte Bett so konfiguriert ist, dass der Tisch und der Roboterarm nicht miteinander in Kontakt kommen, wenn der Roboterarm den Tisch dreht, während der Tisch parallel zu einer horizontalen Ebene in einem Zustand gehalten wird, in dem der Roboterarm eine beliebige Stellung einnimmt, und
das robotisierte Bett so konfiguriert ist, dass es eine Stellung einnimmt, in der der Roboterarm in seiner Gesamtheit unter dem Tisch verborgen ist, wenn er von vertikal oben betrachtet wird.

2. Robotisiertes Bett nach Anspruch 1, wobei
der Roboterarm weiter eine Basis (121) einschließt, die niedriger ist als eine untere Fläche des Tisches, wenn sich das distale Ende des Roboterarms in einer untersten Position befindet und der Tisch in einer Stellung parallel zur horizontalen Ebene ist.

3. Robotisiertes Bett nach Anspruch 2, wobei
das erste bewegliche Element und die Basis durch ein vertikal gerade verlaufendes Gelenk (131) miteinander gekoppelt sind.

4. Robotisiertes Bett nach einem der Ansprüche 1-3, wobei
das distale Ende des Roboterarms einen Endabschnitt oder einen Mittelabschnitt des Tisches trägt.

5. Robotisiertes Bett nach einem der Ansprüche 1-4, wobei
der Tisch einen Tischverschiebemechanismus (2309) aufweist.

6. Robotisiertes Bett nach Anspruch 5, wobei
der Tischverschiebemechanismus so gesteuert und konfiguriert ist, dass der Tisch durch Betätigen eines Stellgliedes in einer Richtung parallel zur horizontalen Ebene verschoben wird oder durch menschliche Kraft verschoben wird.

7. Robotisiertes Bett nach einem der Ansprüche 1-6, umfassend:
einen Abstandssensor (173), der einen Bewegungsbereich des Tisches oder des Roboterarms abtastet, wobei
die Bewegung des Roboterarms angehalten wird, wenn der Abstandssensor ein Objekt im Bewegungsbereich erfasst.

8. Robotisiertes Bett nach einem der Ansprüche 1-7, wobei
das robotisierte Bett so konfiguriert ist, dass es einen Sollpunkt nachverfolgt, und wenn eine Ist-Position aufgrund einer Verformung des Tisches vom Sollpunkt abweicht, eine Haltung des Roboterarms geändert wird, um eine Korrektur der Verlagerung vorzunehmen und die Ist-Position zum Sollpunkt zu bringen.

9. Robotisiertes Bett nach einem der Ansprüche 1-8, umfassend:
elektromagnetische Bremsen (161-166), von denen eine Bremsfunktion eingeschaltet ist, wenn einem beliebigen von einem Stellglied, das die beiden beweglichen Elemente betätigt, die sich um das Drehgelenk drehen, das die beiden beweglichen Elemente koppelt, und einem Stellglied, das den Tisch betätigt, kein Antriebsstrom zugeführt wird, und von denen die Bremsfunktion ausgeschaltet ist, wenn den Stellgliedern Antriebsstrom zugeführt wird, wobei
wobei mindestens eine der elektromagnetischen Bremsen so konfiguriert ist, dass die Bremsfunktion manuell abgeschaltet werden kann, wenn der Antriebsstrom nicht zugeführt wird.

10. Robotisiertes Bett nach einem der Ansprüche 1-9, wobei
der Roboterarm weiter ein drittes bewegliches Element (124) umfasst, das mit dem zweiten beweglichen Element durch ein zweites horizontal drehbares Gelenk (133) gekoppelt ist.

11. Robotisiertes Bett nach Anspruch 10, wobei
der Roboterarm weiter ein viertes bewegliches Element (125) umfasst, das mit einem Drehgelenk (135) gekoppelt ist, das so konfiguriert ist, dass es sich in einer Richtung orthogonal zu einer Längsrichtung des dritten beweglichen Elements dreht.

12. Robotisiertes Bett nach einem der Ansprüche 1-11, wobei
der Roboterarm so konfiguriert ist, dass er den Tisch in mehrere vorbestimmte Positionen bewegt, einschließlich einer Behandlungsposition, in der ein Arzt den Patienten behandelt, und einer Bildgebungsposition, in der eine Bildgebungsvorrichtung den Patienten abbildet.

13. Robotisiertes Bett nach Anspruch 12, wobei
die Bildgebungsvorrichtung eine MRI-Einrichtung (414) oder eine Angiographie-Einrichtung (15) ist.

## Revendications

1. Lit robotisé comprenant :
un bras robotisé (101) qui inclut deux éléments mobiles, un premier élément mobile (122) et un deuxième élément mobile (123), couplés ensemble à l'une de leurs potions d'extrémité par une articulation à rotation horizontale (132) ; et
une table (108), destinée à placer un patient, supportée de manière rotative par une extrémité distale du bras robotisé, dans lequel
le lit robotisé est configuré de sorte que la table et le bras robotisé n'entrent pas en contact l'un avec l'autre, lorsque le bras robotisé entraîne la rotation de la table en même temps que la table est maintenue parallèle à un plan horizontal dans un état dans lequel le bras robotisé adopte une orientation arbitraire, et
le lit robotisé est configuré pour adopter une orientation dans laquelle le bras robotisé est intégralement caché sous la table lorsqu'il est vu verticalement du dessus.

2. Lit robotisé selon la revendication 1, dans lequel
le bras robotisé inclut en outre une base (121) qui est plus basse en hauteur qu'une surface inférieure de la table, lorsque l'extrémité distale du bras robotisé est située en une position la plus basse et la table se trouve dans une orientation parallèle au plan horizontal.

3. Lit robotisé selon la revendication 2, dans lequel
le premier élément mobile et la base sont couplés ensemble par une articulation (131) se déplaçant selon une trajectoire verticalement rectiligne.

4. Lit robotisé selon l'une quelconque des revendications 1-3, dans lequel
l'extrémité distale du bras robotisé supporte une portion d'extrémité ou une portion intermédiaire de la table.

5. Lit robotisé selon l'une quelconque des revendications 1-4, dans lequel
la table présente un mécanisme de coulissement de table (2309).

6. Lit robotisé selon la revendication 5, dans lequel
le mécanisme de coulissement de table est commandé et configuré de sorte que la table soit coulissée dans une direction parallèle au plan horizontal par actionnement d'un actionneur ou soit coulissée par la force humaine.

7. Lit robotisé selon l'une quelconque des revendications 1-6, comprenant :
un capteur de distance (173) qui balaye une plage de mouvement de la table et du bras robotisé, dans lequel
lorsque le capteur de distance détecte un objet dans la plage de mouvement, le mouvement du bras robotisé est arrêté.

8. Lit robotisé selon l'une quelconque des revendications 1-7, dans lequel
le lit robotisé est configuré pour suivre un point cible, et si une position courante est décalée du point cible en raison d'une déformation de la table, une orientation du bras robotisé est modifiée pour apporter une correction au décalage et rapprocher la position courante du point cible.

9. Lit robotisé selon l'une quelconque des revendications 1-8, comprenant :
des freins électromagnétiques (161-166) dont une fonction de freinage est activée en l'absence de courant d'attaque fourni à l'un quelconque parmi un actionneur qui commande les deux éléments mobiles en rotation autour de l'articulation rotative qui couple les deux éléments mobiles et un actionneur qui commande la table, et dont la fonction de freinage est désactivée quand le courant d'attaque est fourni aux actionneurs, dans lequel
au moins l'un des freins électromagnétiques est configuré de sorte que la fonction de freinage soit apte à être désactivée manuellement quand le courant d'attaque n'est pas fourni.

10. Lit robotisé selon l'une quelconque des revendications 1-9, dans lequel
le bras robotisé comprend en outre un troisième élément mobile (124) couplé au deuxième élément mobile par une seconde articulation à rotation horizontale (133).

11. Lit robotisé selon la revendication 10, dans lequel
le bras robotisé comprend en outre un quatrième élément mobile (125) couplé à une articulation rotative (135), qui est configuré pour effectuer une rotation dans une direction perpendiculaire à une direction longitudinale du troisième élément mobile.

12. Lit robotisé selon les revendications 1-11, dans lequel
le bras robotisé est configuré pour déplacer la table vers une pluralité de positions prédéterminées incluant une position de traitement dans laquelle un médecin traite le patient et une position d'imagerie dans laquelle un dispositif d'imagerie capture des images du patient.

13. Lit robotisé selon la revendication 12, dans lequel
le dispositif d'imagerie est un appareil d'IRM (414) ou un appareil angiographique (15).
